# EUROPEAN PATENT APPLICATION

(11) **EP 3 476 944 A1**
(43) Date of publication of application: **01.05.2019**
(21) Application number: 17815522.2
(22) Date of filing: 23.06.2017
(51) Int. Cl.: C12Q 1/68, C12N 15/09

(54) **ONE-STEP REVERSE TRANSCRIPTION TEMPLATE-SWITCHING PCR**

(30) Priority: 23.06.2016 JP 2016125007
(71) Applicant: Riken, Wako-shi, Saitama 351-0198 (JP)
(72) Inventor: SHIROGUCHI Katsuyuki, Wako-shi Saitama 351-0198 (JP)
(74) Representative: Uexküll & Stolberg
(86) International application number: PCT/JP2017/023254
(87) International publication number: WO 2017/222057

(57) **Abstract**

The present invention provides technology for carrying out one-step reverse transcription template-switching PCR more quickly, more easily, and with high specificity. The present invention provides a nucleic acid amplification method for amplifying at least a partial region of RNA using a modified oligonucleotide primer, said nucleic acid amplification method being characterized by the following: a nucleic acid amplification reaction comprises a reverse transcription step a) in which RNA is used as a template, a template switching step b) in which a template-switching oligonucleotide is added to cDNA synthesized in step a), and a DNA amplification step c) in which DNA amplification is carried out by PCR in which the template-switch cDNA synthesized in step b) is used as a template; the steps a) to c) are performed in a single stage in the same reaction system; as a result of being modified, some or all of the primer function of the modified oligonucleotide primer is blocked in the reverse transcription step a); and blocking of the primer function is cancelled in the DNA amplification step c).

SEQUENCE LISTING

<110>  RIKEN

<120>  One-step reverse transcription template switch PCR

<130>  EC004PCT

<150>  JP 2016-125007
<151>  2016-06-23

<160>  5

<170>  PatentIn version 3.5

<210>  1
<211>  30
<212>  DNA
<213>  Artificial Sequence

<220>
<223>  Block Primer in Example 1-5

<400>  1
gagggtagcc ttttgtttgt ttgcaatctc                                        30


<210>  2
<211>  34
<212>  DNA
<213>  Artificial Sequence

<220>
<223>  RT Primer in Example 1-5

<400>  2
aagcacacga gggtagcctt ttgtttgttt gcaa                                   34


<210>  3
<211>  30
<212>  DNA
<213>  Artificial Sequence

<220>
<223>  Template switch Oligo

<400>  3
aagcagtggt atacccgcag agtacatggg                                        30


<210>  4
<211>  33
<212>  DNA
<213>  artificial sequence

<220>
<223>  Block Primer in Example 6

<400>  4
gaggatcttt taactggtac acagcaggtt ctg                                    33

<210>  5
<211>  20
<212>  DNA
<213>  artificial sequence

<220>
<223>  RT Primer in Example 6

<400>  5
cggtgaacag gcagagggtg                                                    20

## Description

### [Technical Field]

The present invention relates to a technology for performing reverse transcription template switching PCR in one step.

### [Background Art]

A reverse transcription polymerase chain reaction (RT-PCR) is universally used in the field of genetic engineering as a method of amplifying a specific gene using RNA as a template. In RT-PCR, a reverse transcriptase (RNA dependent DNA polymerase) is used for reverse transcription of RNA into cDNA, and then the cDNA is amplified to a detectable level by a heat resistant DNA polymerase. While this reaction combination is conventionally performed in two steps (uses a separate tube for each reaction and continuously reacted), technologies are in development for performing this reaction combination in one step (successively reacted in a single tube) by improvement in reverse transcriptase or reaction solution composition.

Template switching is a technique that enables RT-PCR amplification using an RNA as a template, even if the sequence of the 5' terminus of the template RNA is unknown or lacks a common sequence. Template switching utilizes a phenomenon, where a short specific sequence (e.g., a short cytosine rich sequence for Moloney Murine Leukemia Virus derived reverse transcriptase (MMLV RT)) is automatically added to the 3' terminus of a newly synthesized cDNA by the terminal transferase activity of a reverse transcriptase when the reverse transcriptase reaches the 5' terminus of a template RNA. If a sequence that is complementary to this short added sequence is added into the system upon reverse transcription of an oligonucleotide (template switching oligonucleotide) added to the 3' terminus of an anchor sequence, the template switching oligonucleotide hybridizes to the 3' terminus of the synthesized cDNA to extend the template for a reverse transcriptase. Since a reverse transcriptase switches templates and continues cDNA synthesis to the 5' terminus of the anchor sequence, a sequence that is complementary to the anchor sequence is added to the 3' terminus of the cDNA. By using an oligo DNA comprising a sequence that is complementary to a specific sequence in a template RNA or an oligo DNA with a specific known sequence added to the 5' terminus as a reverse transcription primer, a newly synthesized cDNA would also have a known sequence at the 5' terminus. As a result, the newly synthesized cDNA (antisense strand) would comprise a known sequence on both the 5' terminus and the 3' terminus. Therefore, use of a primer set that is designed based on these known sequences enable PCR amplification.

Synthesis of a cDNA corresponding to an mRNA with a poly-(A) tail is achieved by reverse transcription using a random primer or an oligo (dT) containing primer that is complementary to the poly-(A) tail. In this reaction, a cDNA is synthesized from all mRNAs with a poly-(A) tail, so that a cDNA library is constructed. Meanwhile, only a cDNA of a specific gene can be specifically synthesized by using a primer that is specific to a specific gene in reverse transcription.

There is a need for a technology for performing a faster and simpler reverse transcription template switching PCR with higher specificity so that the PCR can be applied for a high throughput operation.

Meanwhile, various hot start technologies have been developed as a technology for avoiding side reactions in PCR. In other words, a PCR reaction mixture is exposed to a temperature from room temperature to 50°C from the preparation of a reaction solution until the temperature of a thermal cycler increases in PCR. Since Tm of a primer is generally set at 50° C or higher, the specificity of the primer is not sufficiently exhibited in this temperature range. Meanwhile, a polymerase exhibits activity (albeit weak activity) in this temperature range, resulting in extension from a mis-annealed primer to cause various side reactions (primer dimers, extra band, or the like). An oligonucleotide primer bound to a thermolabile modifying group is in development as a technology for avoiding such a side reaction (Patent Literatures 1 and 2, and Non Patent Literatures 1 and 2). In this oligonucleotide primer, a 3' terminus hydroxyl group or one or more internucleotide bonds is replaced with a modifying group. Protection by this modifying group suppresses DNA polymerase mediated oligonucleotide primer extension before the first high temperature incubation period in PCR amplification. Due to the presence of a modifying group, a primer is inactive until reaching the first denaturation temperature (in most cases 95° C). After reaching the first denaturation temperature, the modifying group leaves, resulting in a corresponding unmodified oligonucleotide primer that is capable of extending by a polymerase.

### [Citation List]

### [Patent Literature]

[PTL 1] US Patent No. 8133669
[PTL 2] US Patent No. 8361753

### [Non Patent Literature]

[NPL 1] Curr Protoc Nucleic Acid Chem. 2009 Sep; Chapter 4: Unit 4.35 1-17
[NPL 2] Nucleic Acids Res. 2008 Nov; 36(20): e131

### [Summary of Invention]

### [Solution to Problem]

The present invention provides a technology for performing a faster and simpler reverse transcription template switching PCR with high specificity.

The present invention is described hereinafter in more detail. The inventors used a primer that is specific to a specific gene as a reverse transcription primer and used a combination of an oligonucleotide having an anchor sequence in a template switching primer and the reverse transcription primer as a primer set for PCR in reverse transcription template switching PCR to perform a reaction combination of reverse transcription and PCR in one step (one stage in the same reaction system). However, a side reaction is induced in this method. In particular, this tendency was prominent when the number of copies of a template RNA was low and the number of PCR cycles was high. The possible causes of side reactions include: the specificity of PCR is dependent only on the reverse transcription primer; and since reverse transcription primers are in significant excess relative to the number of copies of the template RNA, reverse transcription primers hybridize non-specifically to the template RNA, resulting in non-specific reverse transcription.

In this regard, a reverse transcription primer is used as a primer in not only reverse transcription but also PCR in the reaction combination. Meanwhile, this has been revised to use a primer inactivated by protecting the 3' terminus OH of an oligonucleotide that is the same as the reverse transcription primer with a thermolabile modifying group (hereinafter, an inactivated primer is also referred to as a block primer) as a primer for PCR and to reduce the amount of reverse transcription primer added to successfully suppress side reactions. According to this method, a reverse transcription primer and a block primer hybridize with a template RNA in reverse transcription. Meanwhile, the block primer cannot contribute to reverse transcription due to the protection by the modifying group, so that reverse transcription starts only from the reverse transcription primer. The block primer, even if it hybridizes non-specifically to a template RNA, does not produce a reverse transcription product. When the modifying group leaves the block primer to be converted to an unmodified primer by the first high temperature incubation in PCR amplification, the primer can contribute to an extension reaction, so that PCR progresses. Surprisingly, PCR amplification with high specificity can be achieved even by performing reverse transcription template switching PCR in one step by adding only a block primer without adding an unmodified reverse transcription primer.

The inventors have completed the present invention after further research based on such findings.

In other words, the present invention includes the following:
[1] A method for amplifying a nucleic acid which amplifies at least a part of a region of an RNA using a modified oligonucleotide primer,
   wherein an amplification reaction of the nucleic acid consists of a reverse transcription step a) using the RNA as a template, a template switching step b) for adding a template switching oligonucleotide to a cDNA synthesized in step a), and a DNA amplifying step c) by a PCR using a template switch cDNA synthesized in step b) as a template, wherein steps a) to c) are performed in one stage in the same reaction system,
   wherein the modified oligonucleotide primer is characterized in that due to the modification, a primer function is partially or completely blocked in reverse transcription step a), and blocking of the primer function is cleared in DNA amplification step c).
[2] A method for amplifying a nucleic acid which amplifies at least a part of a region of an RNA using a modified oligonucleotide primer, comprising
   1) providing a composition comprising all reagents (excluding oligonucleotide primers that initiate reverse transcription) required for template switching reverse transcription of a template RNA to a cDNA and for PCR amplification of at least a part of the cDNA, including i) a template switching oligonucleotide, ii) a primer set consisting of a 5' anchor oligonucleotide primer comprising at least a part of an anchor sequence comprised in the template switching oligonucleotide, and a modified oligonucleotide primer, and iii) the template RNA;
   2) incubating the composition provided in 1) at a temperature where reverse transcription can progress, thereby generating a cDNA with a nucleotide sequence that is complementary to the anchor sequence added to a 3' terminus from the template RNA and obtaining a reaction mixture comprising the cDNA; and
   3) subjecting the reaction mixture obtained in 2) to a plurality of rounds of a thermocycling protocol with which PCR can progress, thereby obtaining a nucleic acid with a region sandwiched by the primer set amplified using the cDNA as a template;
   wherein the modified oligonucleotide primer has a primer function in reverse transcription that is partially or completely blocked by the modification, and blocking of the primer function is cleared (cleared) as a result of the reverse transcription or by initial thermal denaturation of PCR.
[3] The method of [2], wherein the composition provided by 1) further comprises an oligonucleotide primer that initiates reverse transcription.
[4] The method of any one of [1] to [3], wherein the modified oligonucleotide primer has one or more complementary regions on a sequence of the same modified oligonucleotide primer, and has a turn structure by the complementary regions or comprises a thermolabile modifying group before initial thermal denaturation of PCR.
[5] The method of [4], wherein the modified oligonucleotide primer comprises a nucleotide sequence that is complementary to a partial sequence of the template RNA.
[6] The method of [5], wherein a part of the modified oligonucleotide primer whose primer function has not been blocked functions as an oligonucleotide primer that initiates reverse transcription by hybridizing to the template RNA.
[7] The method of any one of [1] to [6], wherein a concentration of an oligonucleotide primer that initiates reverse transcription is 40 nM or less.
[8] The method of any one of [1] to [7], wherein a number of rounds of thermal cycling of PCR is 40 or greater.
[9] A kit for performing one-step reverse transcription template switching PCR, comprising:
   i) a template switching oligonucleotide; and
   ii) a primer set consisting of a 5' anchor oligonucleotide primer comprising at least a part of an anchor sequence comprised in the template switching oligonucleotide, and a modified oligonucleotide primer;
   wherein the modified oligonucleotide primer has a primer function in reverse transcription that is partially or completely blocked by the modification, and a primer function in PCR using a product of the reverse transcription as a template is acquired as a result of the reverse transcription or by initial thermal denaturation.
[10] The kit of [9], comprising the oligonucleotide of i) and the primer set of ii) as a composition comprising a mixture thereof.
[11] The kit of [9] or [10], further comprising an oligonucleotide primer that initiates reverse transcription.
[12] The kit of [9] or [10], which does not comprise an oligonucleotide primer that initiates reverse transcription.
   [A1] A method of amplifying at least a part of a region of a target RNA, the method comprising the steps of:
      a) mixing the target RNA, a reagent required for reverse transcription, a reagent required for template switching, and a reagent required for a polymerase chain reaction and subjecting the mixture to a condition under which reverse transcription occurs to provide a cDNA comprising a nucleic acid sequence corresponding to the target RNA and a template switching oligonucleotide; and
      b) subjecting the cDNA obtained in step a) to a condition under which a polymerase chain reaction occurs to amplify at least a part of a region of the cDNA;
      wherein the reagent required for a polymerase chain reaction comprises a modified oligonucleotide primer designed to have a primer function that is partially or completely blocked in step a) and designed to have blocking of the primer function cleared in step b).
   [A2] A method of producing a nucleic acid sample that is amplified based on at least a part of a region of a target RNA, the method comprising the steps of:
      a) mixing the target RNA, a reagent required for reverse transcription, a reagent required for template switching, and a reagent required for a polymerase chain reaction and subjecting the mixture to a condition under which reverse transcription occurs to provide a cDNA comprising a nucleic acid sequence corresponding to the target RNA and a template switching oligonucleotide; and
      b) subjecting the cDNA obtained in step a) to a condition under which a polymerase chain reaction occurs;
      wherein the reagent required for a polymerase chain reaction comprises a modified oligonucleotide primer designed to have a primer function that is partially or completely blocked in step a) and designed to have blocking of the primer function cleared in step b).
   [A3] The method of [A1] or [A2], wherein the reagent required for a polymerase chain reaction optionally comprises a 5' anchor oligonucleotide primer comprising at least a part of an anchor sequence comprised in the template switching oligonucleotide.
   [A4] The method of [A3], wherein the reagent required for a polymerase chain reaction does not comprise the 5' anchor oligonucleotide primer.
   [A5] The method of any one of [A1] to [A4], wherein the reagent required for reverse transcription comprises an oligonucleotide primer that initiates reverse transcription, and the oligonucleotide primer that initiates reverse transcription is comprised in the mixture at a final concentration of about 40 nM or less, or at a mole ratio of about 1:10 or less relative to the modified oligonucleotide primer.
   [A6] The method of any one of [A1] to [A5], wherein the modified oligonucleotide primer has one or more complementary regions on a sequence of the same modified oligonucleotide primer, and has a turn structure by the complementary regions or comprises a thermolabile modifying group before initial thermal denaturation of PCR.
   [A7] The method of any one of [A1] to [A6], wherein the modified oligonucleotide primer comprises a nucleotide sequence that is complementary to a partial sequence of a template RNA.
   [A8] The method of [A7], wherein a part of the modified oligonucleotide primer whose primer function has not been blocked functions as an oligonucleotide primer that initiates reverse transcription by hybridizing to the template RNA.
   [A9] A kit for amplifying at least a part of a region of a target RNA, the kit comprising:
      i) a reagent required for reverse transcription;
      ii) a reagent required for template switching;
      iii) a reagent required for a polymerase chain reaction using a modified oligonucleotide primer; and
      iv) optionally a user manual;
      characterized in that the reagents of i) to iii) and the modified oligonucleotide primer are all mixed in a reaction system as of the initiation of a reaction, wherein the modified oligonucleotide primer is designed to have a primer function that is partially or completely blocked under a condition where reverse transcription occurs and designed to have blocking of the primer function cleared under a condition where a polymerase chain reaction occurs.
   [A10] The kit of [A9], wherein the reagent required for template switching comprises a template switching oligonucleotide, and the reagent required for a polymerase chain reaction optionally comprises a 5' anchor oligonucleotide primer comprising at least a part of an anchor sequence comprised in the template switching oligonucleotide.
   [A11] The kit of [A10], wherein the reagent required for a polymerase chain reaction does not comprise the 5' anchor oligonucleotide primer.
   [A12] The kit of any one of [A9] to [A11], characterized in that the reagent required for reverse transcription comprises an oligonucleotide primer that initiates reverse transcription, and the oligonucleotide primer that initiates reverse transcription is used at a final concentration of about 40 nM or less, or at a mole ratio of about 1:10 or less relative to the modified oligonucleotide primer.
   [A13] The kit of any one of [A9] to [A12], wherein the modified oligonucleotide primer has one or more complementary regions on a sequence of the same modified oligonucleotide primer, and has a turn structure by the complementary regions or comprises a thermolabile modifying group before initial thermal denaturation of PCR.
   [A14] The kit of any one of [A9] to [A13], wherein the modified oligonucleotide primer comprises a nucleotide sequence that is complementary to a partial sequence of a template RNA.
   [A15] The kit of [A14], wherein a part of the modified oligonucleotide whose primer function has not been blocked functions as an oligonucleotide primer that initiates reverse transcription by hybridizing to the template RNA.
   [A16] A composition for amplifying at least a part of a region of a target RNA, comprising a modified oligonucleotide primer, wherein the modified oligonucleotide primer is designed to have a primer function that is partially blocked under a condition where reverse transcription occurs and designed to have the blocking of the primer function cleared under a condition where a polymerase chain reaction occurs, wherein a part of the modified oligonucleotide primer whose primer function has not been blocked functions as an oligonucleotide primer that initiates reverse transcription by hybridizing to a template RNA.
   [A17] The composition of [A16], wherein the modified oligonucleotide primer has one or more complementary regions on a sequence of the same modified oligonucleotide primer, and has a turn structure by the complementary regions or comprises a thermolabile modifying group before initial thermal denaturation of PCR.
   [A18] The composition of [A16] or [A17], wherein the composition is used in one-step reverse transcription template switching PCR.

It is intended that one or more of the aforementioned features can be provided as a combination of one or more of the aforementioned features in addition to as the explicitly shown combinations. Further embodiments and advantages of the present invention are recognized by those skilled in the art by reading and understanding the following Detailed Description as needed.

### [Advantageous Effects of Invention]

According to the present invention, reverse transcription template switching PCR can be expected to be performed in one step with high specificity. In particular, a specific PCR product can be expected to be amplified while suppressing side reactions, even if the number of copies of template RNA is low and the number of PCR cycles is high.

### [Brief Description of Drawings]

Figure **1** shows amplification of a TCRβ chain by one-step reverse transcription template switching PCR under various conditions. The arrow indicates the band of a full length TCRβ chain.
Figure **2** shows amplification of a TCRβ chain by one-step reverse transcription template switching PCR under various conditions. The arrow indicates the band of a full length TCRβ chain.
Figure **3** shows amplification of a TCRβ chain by one-step reverse transcription template switching PCR using a single cell of T cell as a template. The top arrow indicates the band of a full length TCRβ chain. The bottom arrow indicates a band of a TCRβ chain fragment.
Figure **4** shows amplification of a TCRβ chain by one-step reverse transcription template switching PCR under various conditions. The arrow indicates the band of a full length TCRβ chain.
Figure **5** shows amplification of a TCRβ chain by one-step reverse transcription template switching PCR under various conditions. The arrow indicates the band of a full length of a target sequence of a TCRβ chain.
Figure **6** shows amplification of a TCRα chain by one-step reverse transcription template switching PCR using a single cell of T cell as a template. The arrow indicates the band of a full length of a target sequence of a TCRα chain.

### [Description of Embodiments]

The present invention is explained hereinafter. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Thus, unless defined otherwise, all terminologies and scientific technical terms that are used herein have the same meaning as the general understanding of those skilled in the art to which the present invention pertains. In case of a contradiction, the present specification (including the definitions) takes precedence. As used herein, "about" proceeding a numerical value means ± 10% of the subsequent numerical value.

The present invention relates to a method of amplifying at least a part of a region of a template RNA by one-step reverse transcription template switching PCR.

Reverse transcription template switching PCR is a technique that enables RT-PCR amplification using an RNA as a template, even if the sequence of the 5' terminus of the template RNA is unknown or lacks a common sequence. Reverse transcription template switching PCR utilizes a phenomenon, where a short specific sequence is automatically added to the 3' terminus of a newly synthesized cDNA by the terminal transferase activity of a reverse transcriptase when the reverse transcriptase reaches the 5' terminus of a template RNA. For example, a Moloney Murine Leukemia Virus derived reverse transcriptase (MMLV RT) adds a short cytosine rich sequence (e.g., CC, CCC, or CCCC) to the 3' terminus of the synthesized cDNA. If an oligonucleotide (template switching oligonucleotide) comprising a nucleotide sequence with a sequence that is complementary to the short sequence added to the 3' terminus of a specific anchor sequence (first anchor sequence) is added to a system upon reverse transcription, the template switching oligonucleotide hybridizes to the 3' terminus of the synthesized cDNA, via the interaction between the sequence added to the 3' terminus of the cDNA and the complementary sequence of the sequence added to the 3' terminus of the template switching oligonucleotide, to extend the template for a reverse transcriptase. Since a reverse transcriptase, after reaching the 5' terminus of the template RNA, switches a template to a template switching oligonucleotide and continues cDNA synthesis to the 5' terminus thereof, a sequence that is complementary to the anchor sequence (first anchor sequence) of the template switching oligonucleotide is added to the 3' terminus of the cDNA. By using an oligonucleotide primer comprising a sequence that is complementary to a specific sequence in a template RNA or an oligonucleotide primer with a specific anchor sequence (second anchor sequence) added to the 5' terminus (random primer, oligo (dT) primer, or the like) as a revers transcription primer, the newly synthesized cDNA also has a known sequence at the 5' terminus. As a result, the PCR amplification using a newly synthesized cDNA as a template is possible by using a primer set comprising an oligonucleotide primer comprising the known sequence and an oligonucleotide primer comprising at least a part of the first anchor sequence.

In some embodiments, template switching does not need to be performed known the sequence on the 5' terminus side of a template RNA is known.

In the method of the present invention, reverse transcription template switching PCR is performed in "one step (one stage)". "One-step reverse transcription template switching PCR (RT-TS-PCR)" refers to a method for amplifying a nucleic acid from a reverse transcription reaction, characterized by having all reagents required for template switching and PCR mixed as of the initiation of a reaction and advancing a reaction in the same reaction system without adding additional reagents required for reverse transcription, reagents required for template switching, or reagents required for PCR amplification, and preferably without opening the reaction system (e.g., without adding a reagent or opening/closing a tube).

In other words, in the method of the present invention, an amplification reaction of a nucleic acid consists of a reverse transcription step a) using the RNA as a template, a template switching step b) for adding a template switching oligonucleotide to a cDNA synthesized in step a), and a DNA amplifying step c) by a PCR using a template switch cDNA synthesized in step b) as a template, wherein steps a) to c) are performed in one stage in the same reaction system.

In another embodiment, the present invention is a method of amplifying at least a part of a region of a target RNA, the method comprising the steps of: a) mixing the target RNA, a reagent required for reverse transcription, and a reagent required for a polymerase chain reaction and subjecting the mixture to a condition under which reverse transcription occurs, the mixing optionally comprising mixing a reagent required for template switching; and b) subjecting the mixture to a condition under which a polymerase chain reaction occurs to amplify the at least a part of a region of the target RNA; wherein the reagent required for a polymerase chain reaction comprises a modified oligonucleotide primer designed to have a primer function that is partially or completely blocked in step a) and designed to have blocking of the primer function cleared in step b).

Furthermore, the present invention provides a method of producing a nucleic acid sample that is amplified based on at least a part of a region of a target RNA, the method comprising the steps of: a) mixing the target RNA, a reagent required for reverse transcription, and a reagent required for a polymerase chain reaction and subjecting the mixture to a condition under which reverse transcription occurs, the mixing optionally comprising mixing a regent required for template switching; and b) subjecting the mixture to a condition under which a polymerase chain reaction occurs; wherein the reagent required for a polymerase chain reaction comprises a modified oligonucleotide primer designed to have a primer function that is partially or completely blocked in step a) and designed to have blocking of the primer function cleared in step b).

In still another embodiment, the present invention is a method of amplifying at least a part of a region of a target RNA, the method comprising the steps of: a) mixing the target RNA, a reagent required for reverse transcription, a reagent required for template switching, and a reagent required for a polymerase chain reaction and subjecting the mixture to a condition under which reverse transcription occurs to provide a cDNA comprising a nucleic acid sequence corresponding to the target RNA and a template switching oligonucleotide; and b) subjecting the cDNA obtained in step a) to a condition under which a polymerase chain reaction occurs to amplify at least a part of a region of the cDNA; wherein the reagent required for a polymerase chain reaction comprises a modified oligonucleotide primer designed to have a primer function that is partially or completely blocked in step a) and designed to have blocking of the primer function cleared in step b).

In still another embodiment, the present invention is a method of producing a nucleic acid sample that is amplified based on at least a part of a region of a target RNA, the method comprising the steps of: a) mixing the target RNA, a reagent required for reverse transcription, a reagent required for template switching, and a reagent required for a polymerase chain reaction and subjecting the mixture to a condition under which reverse transcription occurs to provide a cDNA comprising a nucleic acid sequence corresponding to the target RNA and a template switching oligonucleotide; and b) subjecting the cDNA obtained in step a) to a condition under which a polymerase chain reaction occurs; wherein the reagent required for a polymerase chain reaction comprises a modified oligonucleotide primer designed to have a primer function that is partially or completely blocked in step a) and designed to have blocking of the primer function cleared in step b).

If the sequence of the 5' terminus of the template RNA is unknown or lacks a common sequence, it is advantageous to perform template switching because a specific anchor sequence can be added to the 5' terminus of the template RNA. On the other hand, if the sequence on the 5' terminus side of the template RNA is known, template switching does not need to be performed.

In one embodiment, the reagent required for template switching can comprise a template switching oligonucleotide. In still another embodiment, a reagent required for a polymerase chain reaction can, but does not need to comprise a 5' anchor oligonucleotide primer comprising at least a part of an anchor sequence comprised in the template switching oligonucleotide. As demonstrated in the Examples herein, a template switching oligonucleotide (TS-Oligo) can also unexpectedly function as a forward primer in PCR amplification. Therefore, a reagent required for a polymerase chain reaction can be free of the 5' anchor oligonucleotide primer or comprise a smaller amount that an amount that is commonly used.

Surprisingly, PCR amplification with high specificity was able to be achieved even by adding only the modified oligonucleotide primer without adding a reverse transcription primer to perform reverse transcription PCR. Although not wishing to be bound by any theory, a part of a modified oligonucleotide primer does not have the function blocked at the time of a reverse transcription reaction, so that a part of the modified oligonucleotide primer whose function is not blocked can function as a reserve transcription primer, or a function of a modified oligonucleotide primer is partially blocked at the time of a reverse transcription reaction, so that the modified oligonucleotide primer whose function is partially blocked can function as a reverse transcription primer in a limited capacity. Therefore in some embodiment, a reagent required for reverse transcription does not need to comprise an oligonucleotide primer that initiates reverse transcription. Even if it is comprised, the oligonucleotide primer that initiates reverse transcription used in the present invention can be contained at a smaller amount than an amount that is commonly used. In some embodiments, the concentration of the oligonucleotide primer that initiates reverse transcription in the composition is, for example, about 40 nM or less, preferably about 20 nM or less, about 10 nM or less, about 2.5 nM or less, about 2.0 nM or less, about 0.63 nM or less, about 0.2 nM or less, about 0.16 nM or less, about 0.02 nM or less, about 2.0 pM or less, about 0.2 pM or less, or about 0.02 pM or less. In another embodiment, the oligonucleotide primer that initiates reverse transcription in the composition is comprised at a mole ratio of about 1:10 or less relative to a modified oligonucleotide primer, preferably about 1:20 or less, about 1:40 or less, about 1:160 or less, about 1:200 or less, about 635:1 or less, about 2000:1 or less, about 2500:1 or less, about 20,000:1 or less, about 200,000:1 or less, about 2,000,000:1 or less, or about 20,000,000:1 or less.

In the method of the present invention, at least one of the oligonucleotide primers in PCR has a primer function in reverse transcription that is partially or completely blocked by a modification, and the blocking of the primer function is cleared in the nucleic acid amplification step of PCR. This can accomplish functional separation of primers that are used in each of the nucleic acid amplification stage of PCR and reverse transcription reaction stage while being in the same reaction system, and is characterized by significant differentiation in primer concentrations at each reaction stage.

Examples of means for blocking/clearing a primer function include the following approaches. 1) a primer function is blocked at the time of reverse transcription by a primer designed to retain a turn structure in the reverse transcription reaction stage or to comprise a thermolabile modifying group.

After a reverse transcription reaction, blocking of a primer function is cleared by detachment of a thermolabile modifying group or dissolution of a turn structure by heat treatment. 2) A primer comprising an artificial base blocks the function as a primer at the reverse transcription reaction stage.

A reverse transcription reaction results in synthesis of a cDNA in which a nucleic acid forming a pair with an artificial base contained in a primer is incorporated from a template RNA by the reverse transcription reaction, and allowing the primer to be annealed to the artificial nucleic acid, thus clearing the blocking of the primer function.

In one-step RT-PCR, all reagents required for reverse transcription of a template RNA into cDNA and all reagents required for PCR using the resulting cDNA as a template are generally included within the reaction system as of the initiation of reverse transcription. Since Tm of a PCR primer is generally set at 50°C or higher, specificity of the primer may not be sufficiently exhibited in a temperature zone where reverse transcription can progress (e.g., 42°C). Further, since the number of copies of a template increases exponentially in PCR, the required PCR primer concentration is dramatically higher than the reverse transcription primer concentration. Therefore, there is a risk of a PCR primer mis-annelaing to a template RNA to cause non-specific reverse transcription due to the mis-annealing as the initiation point and ultimately producing non-specific PCR products in reverse transcription. In the present invention, non-specific reverse transcription can be suppressed by using, as a reverse primer in PCR, a modified oligonucleotide primer, which has a primer function in reverse transcription partially or completely blocked by a modification and has acquired a primer function in PCR using the reverse transcription product as a template as a result of the reverse transcription or by thermal denaturation.

As used herein, "oligonucleotide", "primer", or "oligonucleotide primer" generally refers to a single stranded polynucleotide. This may be naturally-occurring or synthetic. This is generally comprised of a sequence of about 5 to about 50 nucleotides, more preferably about 10 to about 30 nucleotides, or more preferably about 15 to about 25 nucleotides. Oligonucleotides encompass DNA, RNA, and DNA/RNA chimeras.

As used herein, the term "forward primer" refers to an oligonucleotide primer that anneals to an antisense strand when the template RNA in RT-PCR is a sense strand. "Reverse primer" refers to an oligonucleotide primer that anneals to a sense strand.

In one embodiment, the modified oligonucleotide primer used in the present invention comprises a sequence that is complementary to a partial sequence of a template RNA. Although the length of the partial sequence is not particularly limited, the length is generally 10 to 40 bases, preferably 15 to 30 bases, and more preferably 18 to 25 bases. The partially sequence can be a partial sequence of the 3' terminus of a region intended to be amplified in a template RNA. The modified oligonucleotide primer preferably comprises a sequence that is complementary to a partial sequence of a template RNA at the 3' terminus thereof. The modified oligonucleotide primer can comprise a sequence added to the 5' terminus of a sequence that is complementary to a partial sequence of a template RNA. Although the added sequence is not particularly limited, the sequence optimally does not comprise a sequence that is complementary to a partial sequence of a template RNA from the viewpoint of avoiding non-specific hybridization. Examples of the added sequence include specific restriction enzyme recognizing sequences. Although the length of the added sequence is not particularly limited, but shorter sequences are preferred to avoid non-specific hybridization. The length of the added sequence is generally 1 to 50 bases, preferably 1 to 30 bases, and more preferably 1 to 10 bases. In one embodiment, the modified oligonucleotide primer consists of a sequence that is complementary to a partial sequence of a template RNA without an added sequence.

Exemplary embodiments of modifications in the modified oligonucleotide primer used in the present invention include the following:
(1) oligonucleotide primers comprising a thermolabile modifying group;
(2) oligonucleotide primers having one or more complementary regions on a sequence of the same modified oligonucleotide primer and having a turn structure by the complementary regions prior to initial thermal denaturation of PCR to form an intermolecular hairpin loop to exhibit a structure masking a sequence that is complementary to a partial sequence of a template RNA;
(3) oligonucleotide primers comprising an artificial base. Each of the embodiments is discussed in detail below.

### (1) Oligonucleotide primers comprising thermolabile modifying group

In this embodiment, an oligonucleotide primer comprises a thermolabile modifying group so that a modifying nucleotide primer cannot extend the chain along a polynucleotide to which it has hybridized, i.e., cannot extend due to enzyme blocking or a decrease in hybridization to a target nucleic acid. In a preferred embodiment, the 3' terminus hydroxyl group or one or more internucleotide bonds of an oligonucleotide primer is substituted with a thermolabile modifying group. Therefore, a chain does not extend to a substantial degree unless and until a modifying or modified nucleotide is removed. While the modifying group is thermolabile, the group hardly dissociates until reaching the first denaturation temperature in PCR amplification (e.g., about 80 to 105°C, preferably about 85 to 100°C, and more preferably about 90 to 96°C (e.g., 95° C)), so that the primer function is partially or completely blocked in reverse transcription. Once the first denaturation temperature is reached, partial or complete dissociation of a modifying group from a modified oligonucleotide primer is thermally induced. The modified oligonucleotide primer is converted to a corresponding unmodified oligonucleotide primer. An unmodified oligonucleotide primer has an active phosphodiester bond and can extend by polymerase.

Examples of oligonucleotide primers comprising a thermolabile modifying group include the modified oligonucleotide primers with a hydroxyl group at the 3' terminus substituted with a thermolabile modifying group disclosed in US Patent No. 8133669 (the disclosed content is incorporated herein by reference to the same extent as the entirety thereof is explicitly described herein), modified oligonucleotide primers comprising a thermolabile modifying group in one or more internucleotide bonds disclosed in US Patent No. 8361753 (the disclosed content is incorporated herein by reference to the same extent as the entirety thereof is explicitly described herein), and the like.

### (1-1) Modified oligonucleotide primers with a hydroxyl group at the 3' terminus substituted with a thermolabile modifying group (US Patent No. 8133669)

In one embodiment, the modifying group contained at the 3' terminus of the modified oligonucleotide primer is one of the groups selected from the group consisting of 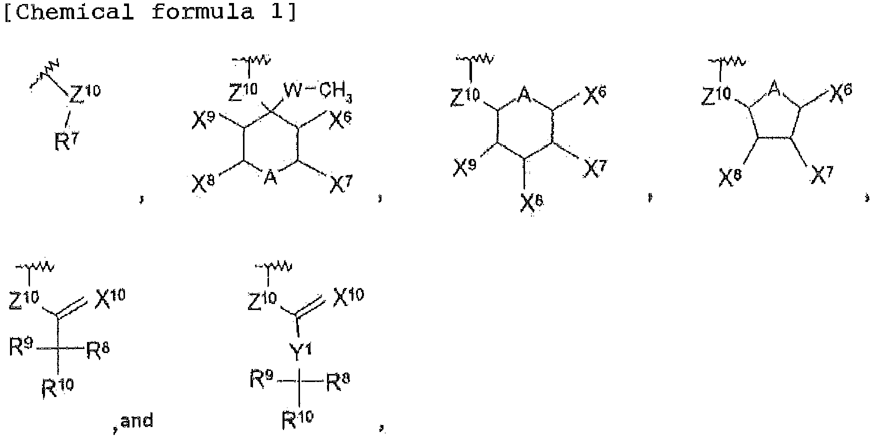 wherein
Z¹⁰ is selected from the group consisting of O, S, and Se;
each R⁷, each R⁸, each R⁹, and each R¹⁰ is independently selected from the group consisting of hydrogen, and a straight or branched optionally substituted hydrocarbyl group having from 1 to 20 carbon atoms, preferably 1 to 10 carbon atoms, and preferably 1 to 6 carbon atoms, wherein
the hydrocarbyl is alkyl, alkenyl, or alkynyl which may include at least one substituent selected from the group consisting of halo, oxo, hydroxyl, alkoxy, amino, amido, cycloalkyl, heterocycloalkyl, aryl, aryloxy, and heteroaryl;
each X⁶, each X⁷, each X⁸, and each X⁹ is independently selected from any substituted or unsubstituted group consisting of acyl, acyloxy, alkenyl, alkenylaryl, alkenylene, alkyl, lower alkyl, alkylene, alkynyl, alkynylaryl, alkoxy, lower alkoxy, alkylaryl, alkylcarbonylamino, alkylsulfinyl, alkylsulfonyl, alkylsulfonylamino, alkylthio, alkynylene, amido, amidino, amino, arylalkynyl, aralkyl, aroyl, arylalkyl, aryl, arylcarbonylamino, arylene, aryloxy, arylsulfonylamino, carbamate, dithiocarbamate, cycloalkenyl, cycloalkyl, cycloalkylene, guanidinyl, halo, halogen, heteroaryl, heteroarylcarbonylamino, heteroaryloxy, heteroarylsulfonylamino, heterocycle, heterocycle, hydrocarbyl, hydrocarbyl, hydrocarbylcarbonyl, hydrocarbyloxycarbonyl, hydrocarbylcarbonyloxy, hydrocarbylene, organosulfinyl, hydroxyl, organosulfinyl, organosulfonyl, sulfinyl, sulfonyl, sulfonylamino, and sulfuryl;
each X¹⁰ is independently selected from the group consisting of O, S, Se, NR¹¹, N-OR¹¹, and CR¹¹R¹²;
each R¹¹ and each R¹² is independently selected from any substituted or unsubstituted group consisting of acyl, acyloxy, alkenyl, alkenylaryl, alkenylene, alkyl, lower alkyl, alkylene, alkynyl, alkynylaryl, alkoxy, lower alkoxy, alkylaryl, alkylcarbonylamino, alkylsulfinyl, alkylsulfonyl, alkylsulfonylamino, alkylthio, alkynylene, amido, amidino, amino, arylalkynyl, aralkyl, aroyl, arylalkyl, aryl, arylcarbonylamino, arylene, aryloxy arylsulfonylamino, carbamate, dithiocarbamate, cycloalkenyl, cycloalkyl, cycloalkylene, guanidinyl, halo, halogen, heteroaryl, heteroarylcarbonylamino, heteroaryloxy, heteroarylsulfonylamino, heterocycle, heterocycle, hydrocarbyl, hydrocarbyl, hydrocarbylcarbonyl, hydrocarbyloxycarbonyl, hydrocarbylcarbonyloxy, hydrocarbylene, organosulfinyl, hydroxyl, organosulfinyl, organosulfonyl, sulfinyl, sulfonyl, sulfonylamino, and sulfuryl; and
each Y¹ is independently selected from the group consisting of O, S, Se, NR⁶, N-OR⁶, and CR⁶R⁷.

In a preferred embodiment, the modifying group is selected from the group consisting of: O-(p-toluene)sulfonate; O-phosphate; O-nitrate; O-[4-methoxy]-tetrahydropyranyl; O-[4-methoxy]-tetrahydrothiopyranyl; O-tetrahydrothiopyranyl; O-[5-methyl]-tetrahydrofuranyl; O-[2-methyl,4-methoxy]-tetrahydropyrany1; O-[5-methyl]-tetrahydropyranyl; O-tetrahydropyranyl; O-tetrahydrofuranyl; O-phenoxyacetyl; O-methoxyacetyl; O-acetyl; O-C(O)-OCH₃; O-C(O)-CH₂CH₂CN; and O-C(S)-OCH₃. In some particularly preferred embodiments, the modifying group is selected from the group consisting of O-methoxytetrahydropyranyl; O-tetrahydropyranyl; and O-tetrahydrofuranyl.

In another embodiment, a modified oligonucleotide primer is a compound represented by formula V 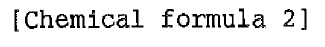 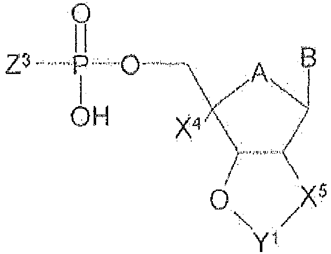 wherein
Z³ is a 3'-O-oligonucleotidyl residue or an oligonucleotide primer;
B is selected from a substituted or non-substituted purine or pyrimidine, any aza or deaza derivative thereof, or any "universal base" or "degenerate base" of any NTP analog which is preferably recognizable by a nucleic acid polymerase;
A is selected from the group consisting of O, S, Se, CR¹R², and NR¹;
each R¹ and each R² is independently selected from the group consisting of H, F, Cl, Br, I, OR³, SR³, NR³R⁴, C(Y)R⁵, substituted or unsubstituted alkyl, alkenyl, alkynyl, aryl, and aralkyl, wherein any substituent may each optionally contain one or more heteroatoms;
each Y is independently selected from the group consisting of O, S, Se, CR¹R², and NR¹;
each R³ and each R⁴ is independently selected from the group consisting of H, substituted or unsubstituted alkyl, substituted or unsubstituted alkenyl, substituted or unsubstituted alkynyl, substituted or unsubstituted aryl, and substituted or unsubstituted aralkyl, wherein any substituent may each optionally contain one or more heteroatoms;
each R⁵ is independently selected from the group consisting of H, F, Cl, Br, OR³, SR³, NR³R⁴, substituted or unsubstituted alkyl, substituted or unsubstituted alkenyl, substituted or unsubstituted alkynyl, substituted or unsubstituted aryl, and substituted or unsubstituted aralkyl, wherein any substituent may each optionally contain one or more heteroatoms;
X⁴ is independently selected from the group consisting of R¹, F, Cl, Br, I, OR³, SR³, SeR³, NR³R⁴, NR³OR³, NR³-NR³R⁴, CN, N₃, C(Y)R⁵, NO₂, CN, and SSR³;
X⁵ is selected from the group consisting of O, S, Se, NR⁶, N-OR⁶, and CR⁶R⁷;
Y¹ is selected from the group consisting of O, S, Se, NR⁶, N-OR⁶, CR⁶R⁷, and C(Y);
each R⁶ and each R⁷ is independently selected from the group consisting of hydrogen, and a straight or branched optionally substituted hydrocarbyl group having from 1 to 20 carbon atoms, preferably 1 to 10 carbon atoms, and preferably 1 to 6 carbon atoms, wherein
the hydrocarbyl is alkyl, alkenyl or alkynyl which may include at least one substituent selected from the group consisting of halo, oxo, hydroxyl, alkoxy, amino, amido, cycloalkyl, heterocycloalkyl, aryl, aryloxy, and heteroaryl; and
X⁵ and Y¹ may each be optionally covalently attached through appropriate atoms or group of atoms to X⁴, X⁵, Z³, A, W, or B portion of the NTP molecule depicted in Formula IB.

In a specific embodiment of formula V, B is thymine, cytosine, adenine, guanine, uracil, aminoallyl-uracil, 7-deazaguanine, 7-deaza-7-methylguanine, 7-deaza-7-iodoguanine, 7-deaza-7-aminoallyl-guanine, 7-deaza-8-azaguanine, 7-deazadenine, 2,6-diaminopurine, 5-nitro-cytosine, 5-aminoallyl-cytosine, 5-(Biotin-16)-cytosine, 5-(Fluorescein-11)-cytosine, 4-methylamino-cytosine, and 2-thio-5-methyluracil, or 4-thio-5-methyluracil.

In a preferred embodiment of formula V, B is adenine, guanine, cytosine, thymine, or uracil.

In a preferred embodiment, a modified oligonucleotide primer is one of the compounds selected from the group consisting of:

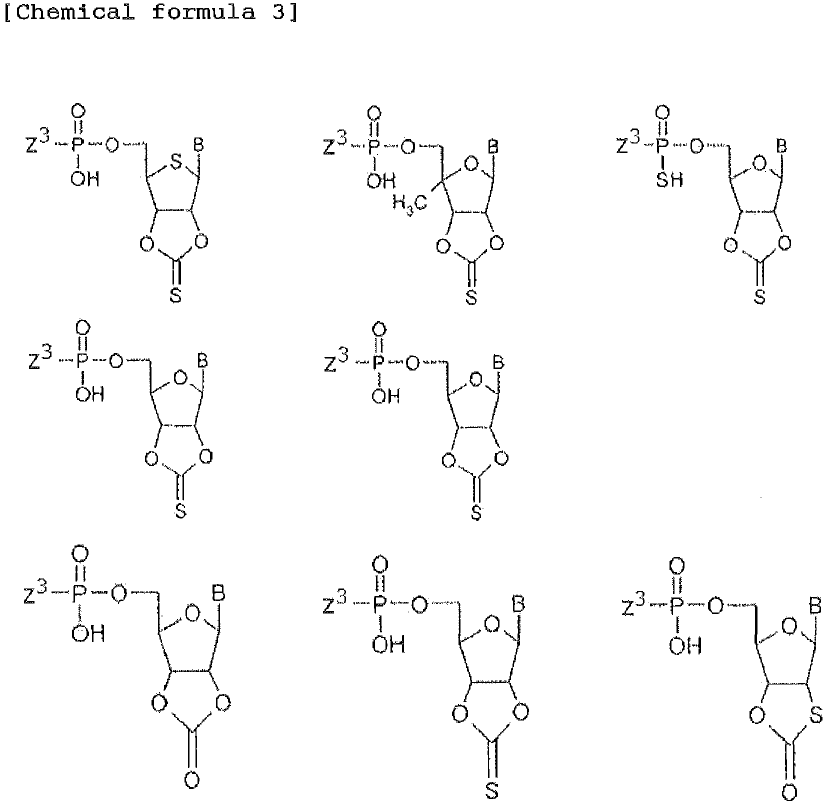

The modified oligonucleotide primer of 1-1 can be manufactured by the method described in US Patent No. 8361753.

### (1-2) Modified oligonucleotide primers comprising a thermolabile modifying group in one or more internucleotide bonds (US Patent No. 8361753)

In one embodiment, a modifying group in the modified oligonucleotide primer comprises a compound of formula I:

[Chemical formula 4] -L-X-R¹

wherein
L is a straight or branched optionally substituted hydrocarbylene group having from 1 to 10 carbon atoms, preferably 2 to 5 carbon atoms, more preferably 3 to 4 carbon atoms, and still more preferably 4 carbon atoms;
X is O, S, S(O), S(O)₂, C(O), C(S), or C(O)NH; and
R¹ is hydrogen or a straight or branched optionally substituted hydrocarbyl group having from 1 to 20 carbon atoms, preferably 1 to 10 carbon atoms, and more preferably 1 to 6 carbon atoms, wherein hydrocarbyl is preferably alkyl, alkenyl or alkynyl which may optionally include at least one substituent selected from the group consisting of halo, oxo, hydroxyl, alkoxy, amino, amido, cycloalkyl, heterocycloalkyl, aryl, aryloxy, and heteroaryl.

In one embodiment, a modifying group provides a compound of formula 1a: 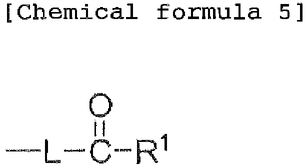 wherein
L is a straight or branched optionally substituted hydrocarbylene group having from 1 to 10 carbon atoms, preferably 2 to 5 carbon atoms, more preferably 3 to 4 carbon atoms, and still more preferably 4 carbon atoms; and
R¹ is hydrogen or a straight or branched optionally substituted hydrocarbyl group having from 1 to 20 carbon atoms, preferably 1 to 10 carbon atoms, and more preferably 1 to 6 carbon atoms, wherein hydrocarbyl is preferably alkyl, alkenyl or alkynyl which may optionally include at least one substituent selected from the group consisting of halo, oxo, hydroxyl, alkoxy, amino, amido, cycloalkyl, heterocycloalkyl, aryl, aryloxy, and heteroaryl.

Preferred embodiments of a modifying group of formula Ia are the following:

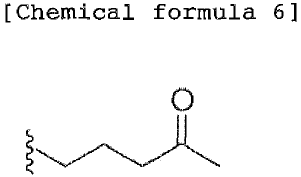

### 4-oxo-1-pentyl

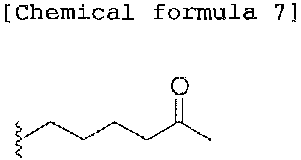

### 5-oxo-1-hexyl

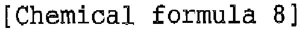

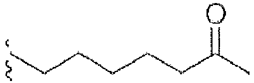

### 6-oxo-1-heptyl

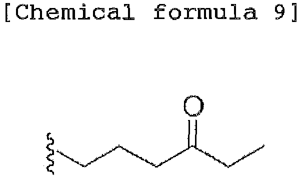

### 4-oxo-1-hexyl

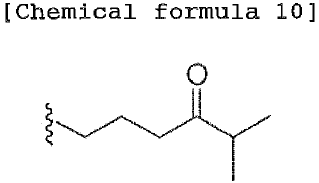

### 5-methyl-4-oxo-1-hexyl

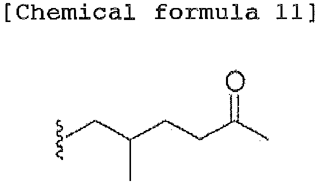

### 2-methyl-5-oxo-hexyl

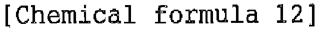

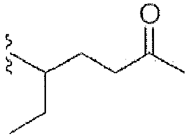

### 1-ethyl-4-oxo-pentyl

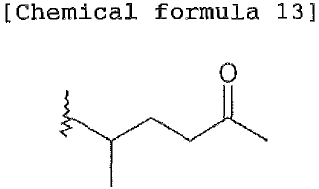

### 1-methyl-4-oxo-pentyl

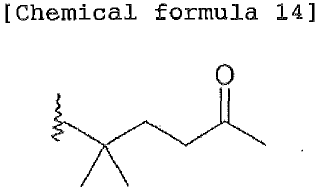

### 1,1-dimethyl-4-oxo-pentyl

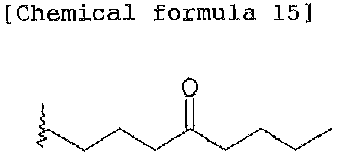

### 4-oxo-1-octyl

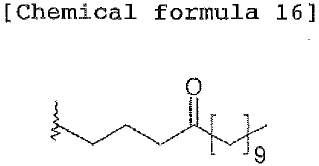

### 4-oxo-1-tetradecyl, and

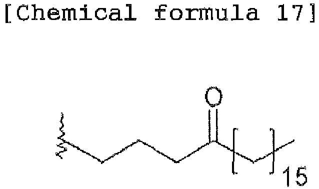

### 4-oxo-1-eicosanyl.

In one embodiment, a modifying group provides a compound of formula Ib:

[Chemical formula 18] -L-S(O)ₖ-R¹

wherein
k is an integer from 0 to 2;
L is a straight or branched optionally substituted hydrocarbylene group having from 1 to 10 carbon atoms, preferably 2 to 5 carbon atoms, more preferably 3 to 4 carbon atoms, and still more preferably 4 carbon atoms; and
R¹ is hydrogen or a straight or branched optionally substituted hydrocarbyl group having from 1 to 20 carbon atoms, preferably 1 to 10 carbon atoms, and more preferably 1 to 6 carbon atoms, wherein hydrocarbyl is preferably alkyl, alkenyl or alkynyl which may optionally include at least one substituent selected from the group consisting of halo, oxo, hydroxyl, alkoxy, amino, amido, cycloalkyl, heterocycloalkyl, aryl, aryloxy, and heteroaryl.

In a preferred embodiment, a modifying group of formula Ib is 4-methylthio-1-butyl described below:

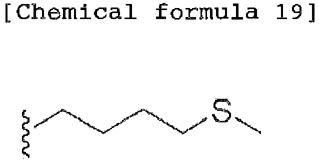

In one embodiment, a modifying group provides a compound of formula Ic: 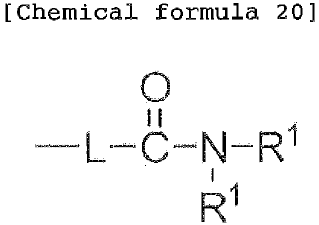 wherein
L is a straight or branched optionally substituted hydrocarbylene group having from 1 to 10 carbon atoms, preferably 2 to 5 carbon atoms, more preferably 3 to 4 carbon atoms, and still more preferably 4 carbon atoms; and
R¹ is hydrogen or a straight or branched optionally substituted hydrocarbyl group having from 1 to 20 carbon atoms, preferably 1 to 10 carbon atoms, and more preferably 1 to 6 carbon atoms, wherein hydrocarbyl is preferably alkyl, alkenyl or alkynyl which may optionally include at least one substituent selected from the group consisting of halo, oxo, hydroxyl, alkoxy, amino, amido, cycloalkyl, heterocycloalkyl, aryl, aryloxy, and heteroaryl.

In a preferred embodiment, a modifying group of formula Ic is 3-(N-tert-butylcarboxamide)-1-propyl described below:

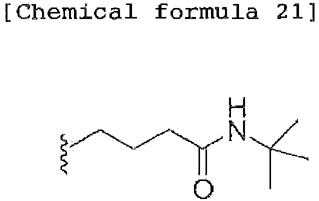

In one embodiment, a modifying group provides a compound of formula Id: 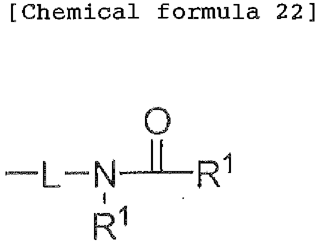 wherein
L is a straight or branched hydrocarbylene group having from 1 to 10 carbon atoms, preferably 2 to 5 carbon atoms, more preferably 3 to 4 carbon atoms, and still more preferably 4 carbon atoms; and
each R¹ is independently hydrogen or a straight or branched optionally substituted hydrocarbyl group having from 1 to 20 carbon atoms, preferably 1 to 10 carbon atoms, and more preferably 1 to 6 carbon atoms, wherein hydrocarbyl is preferably alkyl, alkenyl or alkynyl which may optionally include at least one substituent selected from the group consisting of halo, oxo, hydroxyl, alkoxy, amino, amido, cycloalkyl, heterocycloalkyl, aryl, aryloxy, and heteroaryl.

Examples of a preferred embodiment of a modifying group formula Id includes 2-(N-formyl-N-methyl)aminoethyl and 2-(N-acetyl-N-methyl)aminoethyl (described below):

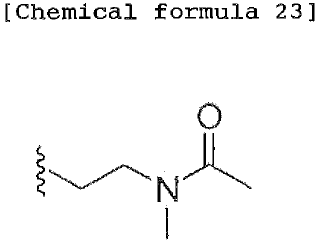

### 2-(N-acetyl-N-methyl)aminoethyl

In another embodiment, a modifying group provides a compound of formula II:

[Chemical formula 24] -L-R²

wherein
L is a straight or branched hydrocarbylene group having from 1 to 10 carbon atoms, preferably 2 to 5 carbon atoms, more preferably 3 to 4 carbon atoms, and still more preferably 4 carbon atoms; and
R² is hydrogen, cyano, or optionally substituted carbocyclic ring, heterocycle, aryl, or heteroaryl having from 5 to 10 atoms.

In a preferred embodiment, a modifying group of formula II is N-(2-hydroxyethyl)-phthalimide described below:

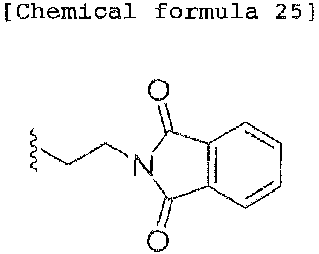

### N-(2-hydroxyethyl)-phthalimide

### In another embodiment, a modifying group provides a compound of formula III:

[Chemical formula 26] -L⁸-A-L^{b}-B

wherein
L^{a} and L^{b} is each independently selected from a single bond or a straight or branched optionally substituted hydrocarbylene group having a single bond or 1 to 8 carbon atoms, preferably 2 to 5 carbon atoms, and more preferably 3 to 4 carbon atoms;
A is O, S, S(O), S(O)₂, Se, CR³R⁴, NR³, C(O), C(S), or CNR³;
B is C(O)R³, C(S)R³, C(O)NR³R⁴, OR³, or SR³;
R³ and R⁴ is each independently hydrogen or a straight or branched optionally substituted hydrocarbyl group having from 1 to 20 carbon atoms, preferably 1 to 10 carbon atoms, and preferably 1 to 6 carbon atoms, wherein hydrocarbyl is preferably alkyl, alkenyl or alkynyl which may optionally include at least one substituent selected from the group consisting of halo, oxo, hydroxyl, alkoxy, amino, amido, cycloalkyl, heterocycloalkyl, aryl, aryloxy, and heteroaryl.

In another embodiment, a modifying group provides a compound of formula IV:

[Chemical formula 27] -L^{a}-O-L^{b}-E-L^{c}-F

wherein
L^{a}, L^{b} , and L^{c} are each independently selected from a single bond or a straight or branched optionally substituted hydrocarbylene group having or 1 to 8 carbon atoms, preferably 2 to 5 carbon atoms, and more preferably 3 to 4 carbon atoms;
D is O, S, S(O), S(O)₂, CR⁵R⁶, or NR⁵;
E is O, S, S(O), S(O)₂, CR⁵R⁶, or NR⁶;
F is hydrogen, C(O)R⁷, C(S)R⁷, C(O)NR⁷R⁸, OR⁷, or SR⁷;
R⁵ and R⁶ are each independently hydrogen, aryl, alkyl, halo, oxo, hydroxyl, alkoxy, aryloxy, or amino, or R⁵ and R⁶ may together form a monocycle or bicycle comprising D, R⁵, R⁶, E and L^{b}, consisting of 5 to 10 atoms, wherein if R⁵ and R⁶ together form a ring, n is from 0 to 2; and
R⁷ and R⁸ are each independently selected from aryl, alkyl, halo, oxo, hydroxyl, alkoxy, aryloxy, amino, amido, optionally substituted cycloalkyl, optionally substituted heterocycloalkyl, optionally substituted aryl, optionally substituted aryloxy, or optionally substituted heteroaryl.

In one embodiment of a compound of formula IV wherein R⁵ and R⁶ together form a ring, a modifying group is methoxymethyl-cyclohexy-1,3-yl-ethyl described below:

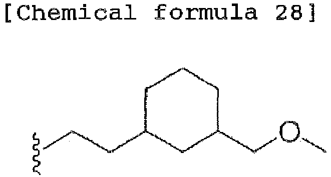

### methoxymethyl-cyclohexy-1,3-yl-ethyl

In one embodiment, a modified oligonucleotide primer has a modified backbone of structure I: 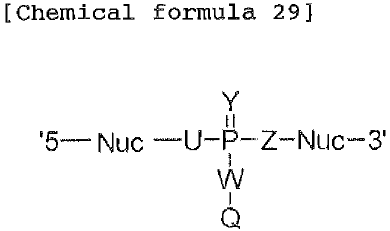 wherein
Nuc is a nucleoside in a primer sequence;
U and Z are independently O, S, Se, NR⁹, or CR⁹R¹⁰;
R⁹ and R¹⁰ are each independently hydrogen or a straight or branched optionally substituted hydrocarbyl having from 1 to 10 carbon atoms; wherein the hydrocarbyl is preferably alkyl, alkenyl or alkynyl which may each include at least one substituent selected from halo, oxo, hydroxyl, alkoxy, aryloxy, amino, amido, or a detectable label;
Y is O, S, or Se;
W is any chemical component that enables Q to be thermally cleaved such as O, S, S(O), S(O)₂, Se, C(O), C(S), C(O)NH, C(N)H, NH, -C(=NR¹¹)-, or NR⁹;
R¹¹ is hydrogen or an optionally substituted hydrocarbyl having 1 to 10 carbon atoms, preferably 1 to 6 carbon atoms, wherein R¹¹ is preferably H, alkyl, or lower alkyl; and
Q is a modifying group comprising one or more thermally cleavable groups.

In one embodiment, modifying group Q comprises one or more thermally cleavable group selected from formulas I, Ia, Ib, Ic, Id, II, III, and IV.

A modified oligonucleotide primer comprises one of the aforementioned modifying groups in at least one internucleotide bonds. A modified oligonucleotide primer preferably comprises one or more of the aforementioned modifying groups at the 3' terminus thereof. A modified oligonucleotide primer preferably comprises one or more of the aforementioned modifying groups in one of the last 6 internucleotide bonds, preferably one of the last three internucleotide bonds, at the 3' terminus thereof.

In another embodiment, an oligonucleotide primer can comprise a sequence with 2, 3, 4, 5, or 6 consecutive modified internucleotide bonds ending at the 3' terminus of the oligonucleotide primer. In still another embodiment, an oligonucleotide primer may comprise a plurality of nonconsecutive 3' modified internucleotide bonds. The 5' terminus of the modified oligonucleotide primer may also have a sequence of a nucleotide comprising a modified internucleotide bond. In yet another embodiment, all internucleotide bonds of an oligonucleotide may be modified.

In another preferred embodiment, a modified oligonucleotide primer comprises a modifying group in a 3' n internucleotide bonds of an oligonucleotide primer, wherein n is an internucleotide bond at the 3' terminus. In yet another embodiment, a modifying group is present in 3' n-1, n-2, n-3, or n-4 internucleotide bond of an oligonucleotide. In yet another embodiment, an oligonucleotide has modifying groups of 2 or more at positions n, n-1, n-2, n-3, n-4, n-5, and n-6; preferably 2 or more at positions n, n-1, n-2, n-3, n-4, n-5, and n-6; preferably 3 or more at positions n, n-1, n-2, n-3, n-4, n-5, and n-6; preferably 4 or more at positions n, n-1, n-2, n-3, n-4, n-5, and n-6; preferably 5 or more at positions n, n-1, n-2, n-3, n-4, n-5, and n-6; or preferably 6 or more at positions n, n-1, n-2, n-3, n-4, n-5, and n-6.

The modified oligonucleotide primer of 1-2 can be manufactured by the method described in US Patent No. 8361753.

### (2) Oligonucleotide primers having one or more complementary regions on a sequence of the same modified oligonucleotide primer and having a turn structure by the complementary regions prior to initial thermal denaturation of PCR to form an intermolecular hairpin loop to exhibit a structure masking a sequence that is complementary to a partial sequence of a template RNA

This embodiment has one or more complementary regions on a sequence of the same modified oligonucleotide primer and has a turn structure by the complementary region prior to initial thermal denaturation processing of PCR to form an intermolecular hairpin loop. The complementary regions refer to a combination of a first sequence comprised of one or more oligonucleotides and a second sequence comprising one or more oligonucleotides that are complementary thereto.

The first and second sequences may be posited adjacent to each other or positioned with one or more oligonucleotides interposed therebetween. If the first sequence or the second sequence comprises a sequence that is complementary to a partial sequence of a template RNA, the sequence that is complementary to the partial sequence of the template RNA is masked by a complementary bond of the first and second sequences. Therefore in such a case, the number of oligonucleotides of the first and second sequences is not particularly limited.

If the first and second sequences do not comprise a sequence that is complementary to a partial sequence of a template RNA, a sequence that is complementary to a partial sequence of a template RNA is comprised between oligonucleotides of the first and second sequences, and the sequence that is complementary to the partial sequence of the template RNA is masked by an intermolecular hairpin loop formation.

Since a sequence that is complementary to a partial sequence of a template RNA is masked, it is unable to hybridize to a corresponding partial sequence of the template RNA upon reverse transcription, so that the primer function is partially or completely blocked. However, since a hairpin loop structure dissociates to expose the sequence that is complementary to the partial sequence of the template RNA at a denaturation temperature in PCR amplification (e.g., about 55 to 105°C, preferably about 85 to 100° C, and more preferably about 90 to 96° C (e.g., 95° C)), the sequence can hybridize to a corresponding partial sequence in a cDNA at a subsequent pairing temperature (i.e., acquires a primer function). The length of the loop portion of the hairpin loop is generally about 5 to 25 bases. The nucleotide sequence of the loop portion is not particularly limited, as long as an intermolecular hairpin loop can be formed.

### (3) Oligonucleotide primers comprising an artificial base

The modified oligonucleotide primer of this embodiment comprises an artificial base (non-naturally occurring base), so that the complementary sequence of a nucleotide sequence of the modified oligonucleotide primer is substantially non-existent in a template RNA (template RNA free of an artificial base). For this reason, hybridization of the modified oligonucleotide primer to the template RNA is suppressed, so that the primer function in reverse transcription would be partially or completely blocked. In one embodiment, 1 or more bases, preferably 3 or more bases, 5 or more bases, 10 or more bases, 12 or more bases, or preferably all 15 bases among the 15 bases at the 3' terminus of the modified oligonucleotide primer are artificial bases. In a preferred embodiment, the base at the most 3' end of the modified oligonucleotide primer is an artificial base.

The modified oligonucleotide primer in this embodiment is used in combination with an oligonucleotide primer for initiating reverse transcription, comprising a partial sequence comprising an artificial base of the modified oligonucleotide primer. The length of the partial sequence comprising an artificial base is 10 to 40 bases, preferably 15 to 30 bases, and more preferably 18 to 25 bases. A partial sequence comprising an artificial base, while not particularly limited, can be for example a partial sequence of the 3' terminus of the modified oligonucleotide primer. The oligonucleotide primer for initiating reverse transcription comprises a sequence that is complementary to a partial sequence of a template RNA and the partial sequence comprising the artificial base, and the partial sequence comprising the artificial base is added to the 5' side of the sequence that is complementary to the partial sequence of the template RNA. The length of the partial sequence of the template RNA is not particularly limited, but is generally 10 to 40 bases, preferably 15 to 30 bases, and more preferably 18 to 25 bases. The partial sequence can be a partial sequence of the 3' terminus of a region intended to be amplified in the template RNA. An oligonucleotide primer for initiating reverse transcription preferably comprises a sequence that is complementary to a partial sequence of a template RNA at the 3' terminus thereof.

When such a combination is used to perform one-step reverse transcription template switching PCR, a cDNA with a partial sequence comprising an artificial base of a modified oligonucleotide primer added to the 5' terminus is synthesized in reverse transcription. The modified oligonucleotide primer comprising an artificial base acquires, as a result thereof, a primer function in PCR using the cDNA as a template. In addition, a region of interest can be specifically modified by PCR amplification using said cDNA as a template and the modified oligonucleotide primer as one of the primers.

Examples of artificial bases include, but are not limited to, Z base/F base (Proc. Natl. Acad. Sci. USA 1997, 94, 105061; Nat. Struct. Biol. 1998, 5, 950; Nat. Struct. Biol. 1998, 5, 954), Q base (J. Am. Chem. Soc. 1999, 121, 2323), iso-G base/iso-C base (J. Am. Chem. Soc. 1989, 111, 8322), 2-thio T (T^{S}) base (Nucleic Acids Res. 2005, 33, 5640), P base/Z base (Nucleic Acids Res. 2007, 35, 4238), PICS base (J. Am. Chem. Soc. 1999, 121, 11585), 5SICS base/MMO2 base/NaM base (J. Am. Chem. Soc. 2009, 131, 14620), 2-amino-6-dimethylaminopurine(x)/2-oxopyridine(y)(Proc. Natl. Acad. Sci. USA 2001, 98, 4922), 2-amino-6-(2-thienyl)purine (s) (J. Am. Chem. Soc. 2005, 127, 17286; Nucleic Acids Res. 2005, 33, e129; Biotechniques 2006, 40, 711), imidazolin-2-one(z) (J. Am. Chem. Soc. 2004, 126, 13298), Ds base/Pa base (Nat. Methods 2006, 3, 729), Pn base (J. Am. Chem. Soc. 2007, 129, 15549), Px base (Nucleic Acids Res. 2009, 37, e14), xA base, xT base (J. Am. Chem. Soc. 2004, 126, 11826), Im-N° base/Na-O^{N} base, Im-O^{N} base/Na-N^{O} base (J. Am. Chem. Soc. 2009, 131, 1644; and Angew. Chem. Int. Ed. 2005, 44, 596), and the like. These artificial bases can contribute to reverse transcription and/or PCR amplification by forming the following base pairs: Z-F base pair, Q-F base pair, isoG-isoC base pair, A-T^{S} base pair, P-Z base pair, PICS-PICS base pair (self-complementary), 5SICS-MMO2 base pair, 5SICS-NaM base pair, x-y base pair, s-y base pair, s-z base pair, Ds-Pa base pair, Ds-Pn base pair, Ds-Px base pair, xA-T base pair, A-xT base pair, Im-N⁰-Na-O^{N} base pair, and Im-O^{N}-Na-N⁰ base pair.

The method of the present invention is described hereinafter in further detail.

The method of the present invention first provides a composition comprising all reagents (excluding oligonucleotide primers that initiate reverse transcription) that are required for template switching reverse transcription of a template RNA into a cDNA, and for PCR amplification of at least a part of the cDNA, including
i) a template switching oligonucleotide,
ii) a primer set consisting of a 5' anchor oligonucleotide primer comprising at least a part of an anchor sequence comprised in the template switching oligonucleotide, and the modified oligonucleotide primer, and
iii) the template RNA.

The template switching oligonucleotide comprises an anchor sequence and a sequence that is complementary to a sequence added to the 3' terminus of a newly synthesized cDNA (also simply referred to as an RT addition sequence) by the terminal transferase activity of the reverse transcriptase when a reverse transcriptase has reached the 5' terminus of the template RNA, and an anchor sequence (first anchor sequence) is added to the 5' terminus of a complementary sequence of the RT addition sequence. Preferably, the complementary sequence of the RT addition sequence is positioned at the 3' terminus of the template switching oligonucleotide. The RT addition sequence is dependent on the type of reverse transcriptase. For example, a Moloney Murine Leukemia Virus derived reverse transcriptase (MMLV RT) adds a short cytosine rich sequence (e.g., CC, CCC, or CCCC) to the 3' terminus of the synthesized cDNA. Thus, a short guanine rich sequence (e.g., GG, GGG, or GGGG), which is the complementary sequence thereof, is comprised in the template switching oligonucleotide as the complement sequence of the RT addition sequence. An anchor sequence refers to an artificial sequence that is added to the 5' terminus of an oligonucleotide. An anchor sequence is preferably a sequence that does not exist in the nature. The length of an anchor sequence is not particularly limited, but is generally about 10 bases to 100 bases, and preferably about 15 bases to about 50 bases.

A template switching oligonucleotide may be a DNA or an RNA, or a DNA/RNA chimera. To efficiently function as a template in reverse transcription, a template switching oligonucleotide is optimally an RNA or a DNA/RNA chimera, and more preferably a DNA/RNA chimera. In one embodiment, a part of a complementary sequence of an RT addition sequence is an RNA, and a part of an anchor sequence is a DNA or a DNA/RNA chimera. A template switching oligonucleotide also functions as the 5' anchor oligonucleotide primer explained below. Therefore, in some embodiments, a 5' anchor oligonucleotide primer can be omitted or added at a small amount. There has been no example of performing reverse transcription template switching and PCR amplification in the same reaction system. The Examples herein are the first to demonstrate that a template switching oligonucleotide functions as a forward primer of PCR amplification.

A 5' anchor oligonucleotide primer comprises a part or all of the anchor sequence (first anchor sequence) comprised in the template switching oligonucleotide. The length of a part or all of the anchor sequence is generally 10 to 40 bases, preferably 15 to 30 bases, and more preferably 18 to 25 bases. The primer is a DNA or a DNA/RNA chimera and preferably a DNA so that it can function as a primer in PCR. A 5' anchor oligonucleotide primer can be a forward primer in PCR.

Examples of a template RNA that can be used include, but are not limited to, mRNA, rRNA, tRNA, non-coding RNA, chemically synthesized RNA, and the like. The mRNA, rRNA, and tRNA may be derived from any cell or tissue. The mRNA, rRNA, and tRNA may be collected from a small amount of cell/tissue (e.g., single cell) obtained by utilizing a cell sorter or the like. The mRNA, rRNA, and tRNA may be in a form contained as a part of a total RNA.

The composition comprises all of the reagents (excluding oligonucleotide primers that initiate reverse transcription) that are required for template switching reverse transcription of the template RNA into a cDNA and for PCR amplification of at least a part of the cDNA. In addition to the aforementioned template switching oligonucleotide, primer set, and template RNA, examples of the reagent include the following.
*Reverse transcriptase (RNA dependent DNA polymerase)
*Heat resistant DNA polymerase (DNA dependent DNA polymerase)
*dNTPs mixture

To form an RT addition sequence to the 3' terminus of a cDNA, a reverse transcriptase that is used has terminal transferase activity. Examples of reverse transcriptases with terminal transferase activity include, but are not limited to, Moloney Murine Leukemia Virus derived reverse transcriptases (MMLV RT). Terminal transcriptase activity is preferably activity of adding a short cytosine rich sequence (e.g., CC, CCC, or CCCC) to the 3' terminus of a synthesized cDNA.

Representative examples of heat resistant DNA polymerases include, but are not limited to, Taq, Tth, KOD, Pfu, Bst, and the like. Various heat resistant DNA polymerases that can be used in PCR have been developed, which can all be used in the present invention. Heat resistant DNA polymerases that can be used in PCR are well known to, and appropriately selectable by, those skilled in the art.

In one embodiment, the composition further comprises an oligonucleotide primer that initiates reverse transcription. An oligonucleotide primer that initiates reverse transcription initiates reverse transcription by hybridizing to a template RNA due to comprising a sequence that is complementary to a partial sequence of a template RNA. The length of the partial sequence is not particularly limited, but is generally 10 to 40 bases, preferably 15 to 30 bases, and more preferably 18 to 25 bases. An oligonucleotide primer that initiates reverse transcription preferably comprises a sequence that is complementary to a partial sequence of a template RNA at the 3' terminus thereof. An anchor sequence (second anchor sequence) may be added to the 5' terminus of a sequence that is complementary to a partial sequence of a template RNA. A second anchor sequence is preferably a sequence that does not exist in nature. The length of a second anchor sequence is not particularly limited, but is generally about 10 bases to 100 bases, and preferably about 15 bases to 50 bases. A second anchor sequence is preferably non-identical to the first anchor sequence. In one embodiment, a second anchor sequence comprises an artificial base. In one embodiment, an oligonucleotide primer that initiates reverse transcription does not comprise a second anchor sequence. An oligonucleotide primer that initiates reverse transcription is a primer that is specific to a specific gene, an oligo dT primer that binds to a poly-A tail of mRNA, or a random primer such as a random hexamer primer, but is preferably a primer that is specific to a specific gene. Said primer comprises a sequence that is complementary to a partial sequence of an RNA (e.g., mRNA) encoding a gene of interest. An oligonucleotide primer that initiates reverse transcription is a DNA or a DNA/RNA chimera and preferably a DNA so that the primer can function as a primer in reverse transcription.

In one embodiment, a region where an oligonucleotide primer that initiates reverse transcription hybridizes and a region where the modified oligonucleotide primer hybridizes on a template RNA at least partially overlap. The length of an overlapping hybridization region is not particularly limited, but is generally 10 bases or greater, preferably 15 bases or greater, and more preferably 18 bases or greater. The length of an overlapping hybridization region can be, for example, 40 bases or less, 30 bases or less, or 25 bases or less.

In a preferred embodiment, the 5' terminus of a region of a template RNA where a modified oligonucleotide primer hybridizes is positioned closer to the 5' side (upstream) of the template RNA than the 5' terminus of a region of the template RNA where an oligonucleotide primer that initiates reverse transcription hybridizes. In other words, both primers are designed so that the 3' terminus of the modified oligonucleotide primer hybridizes with the template RNA closer to the 5' side (upstream) of the template RNA than the 3' terminus of the oligonucleotide primer that initiates reverse transcription. Improvement in the specificity of amplification can be expected by designing the two primers in such a semi-nested positional relationship. In such a case, the region of the template RNA where the oligonucleotide primer that initiates reverse transcription hybridizes and the region of the template RNA where the modified oligonucleotide primer hybridizes may be positioned to partially overlap in a semi-nested form, or positioned in a full-nested form without overlap.

When the region of the template RNA where the oligonucleotide primer that initiates reverse transcription hybridizes partially overlaps the region of the template RNA where the modified oligonucleotide primer hybridizes, both primers are preferably designed so that the 5' terminus of the region of the template RNA where the modified oligonucleotide primer hybridizes is closer to the 5' side (upstream) of the template RNA than the 5' terminus of the region of the template RNA where the oligonucleotide primer that initiates reverse transcription hybridizes by, for example, 1 to 12 bases, preferably 1, 2, 3, 4, or 5 bases (i.e., so that the 3' terminus of the modified oligonucleotide primer hybridizes closer to the 5' side of the template RNA than the 3' terminus of the oligonucleotide primer that initiates reverse transcription by, for example, 1 to 10 bases, and preferably 1, 2, 3, 4 or 5 bases), but the design is not limited thereto.

In another embodiment, a region of a template RNA where an oligonucleotide primer that initiates reverse transcription hybridizes and a region of the template RNA where the modified oligonucleotide primer hybridizes at least partially overlap, and the 5' terminus of the region of the template RNA where the modified oligonucleotide primer hybridizes matches the 5' terminus of region of the template RNA where the oligonucleotide primer that initiates reverse transcription hybridizes. In other words, the 3' terminus of the modified oligonucleotide primer hybridizes with the template RNA at the same position as the 3' terminus of the oligonucleotide primer that initiates reverse transcription.

In one embodiment, a region of a template RNA where an oligonucleotide primer that initiates reverse transcription hybridizes and a region of the template RNA where the modified oligonucleotide primer hybridizes are identical. In this embodiment, the oligonucleotide primer that initiates reverse transcription can be an unmodified oligonucleotide primer corresponding to the modified oligonucleotide primer.

In another embodiment, the modified oligonucleotide primer comprises a partial sequence of an oligonucleotide primer that initiates reverse transcription at the 3' terminus thereof. The length of said partial sequence (hereinafter, also called a common sequence) is not particularly limited, but is generally 10 bases or greater, preferably 15 bases or greater, and more preferably 18 bases or greater. The length of said 3' terminus partial sequence can be, for example, 40 bases or less, 30 bases or less, or 25 bases or less. In one embodiment, said common sequence can be a partial sequence of the 3' terminus of an oligonucleotide primer that initiates reverse transcription. In another embodiment, the 3' terminus of said common sequence is positioned closer to the 5' side than the 3' terminus of the oligonucleotide primer that initiates reverse transcription by at least 1 base (e.g., 1 to 20 bases, 1 to 10 bases, or 1 to 8 bases). In one embodiment, said common sequence is a sequence that is complementary to a partial sequence of a template RNA, or a partial sequence thereof, comprised in an oligonucleotide primer that initiates reverse transcription. In one embodiment, said common sequence is a second anchor sequence or a partial sequence thereof. In one embodiment, said common sequence is a partial sequence of an oligonucleotide primer that initiates reverse transcription, which straddles a sequence that is complementary to a partial sequence of a template RNA and a second anchor sequence. In one embodiment, the modified oligonucleotide primer is an oligonucleotide primer comprising an artificial base, and an oligonucleotide primer that initiates reverse transcription comprises a second anchor sequence comprising an artificial base at the 5' terminus, and a common sequence is a second anchor sequence or a partial sequence thereof.

If the compound comprises an oligonucleotide primer that initiates reverse transcription, the concentration of the oligonucleotide primer may be an amount that is sufficient for initiating reverse transcription. If one copy of a cDNA comprising a region intended to be amplified can be synthesized, this can be amplified to a detectable level by the subsequent PCR. Therefore, the composition (reaction system) only needs to comprise at least one copy, preferably 10 copies or more, and more preferably 100 copies or more of oligonucleotide primer that initiates reverse transcription. If the concentration of the oligonucleotide primer that initiates reverse transcription is too high, side reactions due to non-specific hybridization can be induced. The concentration of the oligonucleotide primer that initiates reverse transcription in the composition is for example about 40 nM or less, preferably about 20 nM or less, about 10 nM or less, about 2.5 nM or less, about 2.0 nM or less, about 0.63 nM or less, about 0.2 nM or less, about 0.16 nM or less, about 0.02 nM or less, about 2.0 pM or less, about 0.2 pM or less, or about 0.02 pM or less.

In another embodiment, the composition does not comprise an oligonucleotide primer that initiates reverse transcription. In this embodiment, an oligonucleotide primer comprising a thermolabile modifying group and a sequence that is complementary to a partial sequence of a template RNA is used as the modified oligonucleotide primer. The modified oligonucleotide primer preferably comprises a thermolabile modifying group at the 3' terminus or one or more internucleotide bonds. A thermolabile modifying group comprised in the modified oligonucleotide primer hardly dissociates until reaching the first denaturation temperature (e.g., about 80 to 105°C, preferably about 85 to 100° C, and more preferably about 90 to 96° C (e.g., 95°C)) in PCR amplification. Meanwhile, the inventors have found that such a thermolabile modifying group slightly dissociates at a temperature where reverse transcription progresses (e.g., 45°C), and a corresponding unmodified oligonucleotide generated as a result thereof can function as an oligonucleotide primer that initiates reverse transcription.

The composition may comprise a buffer, salt (magnesium ion or the like), or RNAase inhibitor as needed.

The concentration of a template switching oligonucleotide comprised in the composition is not particularly limited as long as the method of the present invention can be practiced, but is, for example, about 0.05 to 5.0 µM and preferably 0.1 to 1.0 µM.

The concentration of the modified oligonucleotide primer and 5' anchor oligonucleotide primer comprised in the composition is equivalent to the primer concentration for conventional PCR, such as about 0.1 to 1.0 µM.

The concentration of other constituents (template RNA, reverse transcriptase, heat resistant DNA polymerase, dNTPs mixture, buffer, salt, and RNAase inhibitor) that can be contained in the composition is well known in prior art one-step RT-PCR. The concentration used in the context of the present invention can also be optimized from routine experimentation.

Next, the composition provided above is incubated at a temperature where reverse transcription can progress. A temperature at which reverse transcription can progress can be appropriately adjusted depending on the type of reverse transcriptase, but is generally 37°C to 62° C and preferably 37°C to 55° C. Incubation time can be appropriately adjusted while considering the size of a template RNA or the like, but is generally 30 seconds to 120 minutes and preferably 5 minutes to 60 minutes. With the incubation, an oligonucleotide primer that initiates reverse transcription comprised in the composition or an unmodified oligonucleotide generated by dissociation of a thermolabile modifying group from a modified oligonucleotide primer primes reverse transcription to synthesize a cDNA (antisense strand) that is complementary to a template RNA. A reverse transcriptase, after reaching the 5' terminus of the template RNA, switches the template to a template switching oligonucleotide and continues cDNA synthesis to the 5' end thereof, thus producing a single stranded cDNA (antisense strand) to which a sequence that is complementary to an anchor sequence of the template switching oligonucleotide is added on the 3' end.

Next, a reaction mixture comprising the resulting cDNA is subjected to a plurality of rounds of a thermal cycling protocol with which PCR can progress. A cycle of the thermal cycling protocol is comprised of a three temperature steps, i.e., denaturation (also called thermal denaturation), annealing, and extension. Denaturation is not particularly limited as long as the temperature is sufficient for dissociating a double stranded DNA. The preferred lower limit and upper limit of the thermal denaturation temperature are 90° C and 100°C, respectively. Annealing is a step of annealing a primer to a dissociated DNA. The temperature in this step (annealing temperature) is not particularly limited, but the lower limit of the annealing temperature is preferably 45° C and more preferably 50° C. Meanwhile, the upper limit is preferably 75° C and more preferably 70° C. Extension is a step of synthesizing a complementary strand with a DNA polymerase. The temperature at this time (extension temperature) is not particularly limited, but the lower limit and the upper limit of a preferred extension temperature is 50° C and 80°C, respectively. In this cycle, the annealing temperature does not exceed the extension reaction temperature. The annealing and extension can be performed at one temperature to configure a thermal cycling protocol as a cycle of substantially two temperature steps. In such a case, the lower limit of a temperature for annealing and extension is preferably 50° C and more preferably 55°C. Meanwhile, the upper limit is preferably 70° C and more preferably 65° C. Examples of incubation time in each step include 1 second to 5 minutes, but those skilled in the art can readily determine a suitable incubation time while considering the size of amplification product or the like.

A denaturation step (pre-incubation step) can be performed to inactive a reverse transcriptase before subjecting a reaction mixture to a thermal cycling protocol. The denaturation temperature is not particularly limited as long as a reverse transcriptase can be inactivated, but the lower limit and the upper limit of a preferred thermal denaturation temperature are 90° C and 100°C, respectively. The denaturation time is not particularly limited as long as a reverse transcriptase can be inactivated, but is generally 1 minute to 15 minutes.

If an oligonucleotide primer comprising a thermolabile modifying group is used as a modified oligonucleotide primer, a modifying group is dissociated from a modified oligonucleotide primer and the primer is converted to a corresponding unmodified oligonucleotide primer in the first denaturation step or pre-incubation step of a thermal cycling protocol. An unmodified oligonucleotide primer has an active phosphodiester bond and can prime the extension by a polymerase.

In the first annealing and extension steps of a thermal cycling, a 5' anchor oligonucleotide primer is annealed to a sequence that is complementary to an anchor sequence at the 3' end of a single stranded cDNA (antisense strand) obtained in the reverse transcription step, leading to extension by a polymerase and synthesis of a cDNA (sense strand) in which an anchor sequence (first anchor sequence) is added to the 5' terminus. As a result, a double stranded cDNA in which an anchor sequence is added to the 5' terminus of a sense strand is produced.

In addition, a reaction mixture comprising the double stranded cDNA is subsequently subjected to a plurality of rounds of thermal cycling protocol to amplify a region sandwiched by a 5' anchor oligonucleotide primer and a modified oligonucleotide primer (i.e., from the 5' terminus anchor sequence to the region where the modified oligonucleotide primer hybridizes).

The number of rounds of thermal cycling can be appropriately determined while considering the amount of template RNA or the like, but is for example 20 rounds or more, and preferably 30 rounds or more, 40 rounds or more, 45 rounds or more, 50 rounds or more, or 55 rounds or more. In a common RT-PCR, even with a low number of copies of template RNA (e.g., single copy), an amplification reaction reaches saturation after about 40 rounds of thermal cycling. Meanwhile in the method of the present invention (especially when using a modified oligonucleotide primer comprising a thermolabile modifying group), an amplification reaction does not reach saturation even after 45 rounds or more, 50 rounds or more, or 55 rounds or more of thermal cycling, so that further amplification can be possible. Although not wishing to be bound by any theory, the amplification efficiency per a round of thermal cycle can be more suppressed than common RT-PCR in the method of the present invention (especially when using a modified oligonucleotide primer comprising a thermolabile modifying group). Thus, when the number of copies of a template RNA intended to be amplified is low (e.g., 100 copies or less, 10 copies or less, or a single copy), or when the method of the present invention is performed using an RNA (especially total RNA) isolated from a single cell as a template RNA, the number of rounds of thermal cycling is preferably 40 rounds or more, 45 rounds or more, 50 rounds or more, or 55 rounds or more.

The method of the present invention can be expected to perform reverse transcription template switching PCR with high specificity in one step. The specificity of reverse transcription template switching PCR can be substantially determined only by a reverse primer, but the present invention can amplify a gene of interest with high specificity by employing the aforementioned modified oligonucleotide primer as a reverse primer. Especially when the number of copies of a template RNA is low (e.g., when RNA from a single cell is used as a template), a specific PCR product can be expected to be amplified while minimizing side reactions even when the number of PCR cycles is high. Therefore, a reverse primer that is specific to a constant region of an antigen receptor (e.g., antibody (heavy chain or light chain) or T cell receptor (α chain, β chain, γ chain, or δ chain) can be used as the aforementioned modified oligonucleotide primer to perform sequence analysis of an antigen recognition site of the antigen receptor at a single cell level.

The present invention can also provide a kit for performing one-step reverse transcription template switching PCR, comprising:
i) a template switching oligonucleotide; and
ii) a primer set consisting of a 5' anchor oligonucleotide primer comprising at least a part of an anchor sequence comprised in the template switching oligonucleotide, and a modified oligonucleotide primer, and a modified oligonucleotide primer;
wherein the modified oligonucleotide primer has a primer function in reverse transcription that is partially or completely blocked by the modification, and a primer function in PCR using a product of the reverse transcription as a template is acquired as a result of the reverse transcription or by initial thermal denaturation.

In one embodiment, the kit of the present invention further comprises an oligonucleotide primer that initiates reverse transcription.

In one embodiment, the kit of the present invention does not further comprise an oligonucleotide primer that Initiates reverse transcription.

The kit of the present invention may also comprise other reagents required for performing one-step reverse transcription template switching PCR (e.g., reverse transcriptase (RNA dependent DNA polymerase), heat resistant DNA polymerase (DNA dependent DNA polymerase), dNTPs mixture, buffer, salt (magnesium ion or the like), or RNAase inhibitor).

The reagents may be contained in a single package after being sealed in their respective separate container or provided as a composition comprising a mixture of some or all of the reagents.

In one embodiment, the kit of the present invention comprises the oligonucleotide of i) and the primer set of ii) as a composition comprising a mixture thereof.

In one embodiment, the composition further comprises an oligonucleotide primer that initiates reverse transcription.

In one embodiment, the composition does not further comprise an oligonucleotide primer that initiates reverse transcription.

The composition may comprise 1, 2, 3, 4, 5, or 6 reagents selected from the group consisting of a reverse transcriptase (RNA dependent DNA polymerase), heat resistant DNA polymerase (DNA dependent DNA polymerase), dNTPs mixture, buffer, salt (magnesium ion or the like), and RNAase inhibitor.

If the kit of the present invention is used, one-step reverse transcription template switching PCR can be readily performed with the method of the present invention by using any template RNA.

The definitions of the terms of each constituent comprised in the kit of the present invention are described above in the method of the present invention.

The present invention also provides a kit for amplifying at least a part of a region of a target RNA, the kit comprising: i) a reagent required for reverse transcription; ii) optionally a reagent required for template switching; iii) a reagent required for a polymerase chain reaction using a modified oligonucleotide primer; and iv) optionally a user manual; characterized in that the reagents of i), the reagent of ii) if present, the reagent of iii) and the modified oligonucleotide primer are all mixed in a reaction system as of the initiation of a reaction, wherein the modified oligonucleotide primer is designed to have a primer function that is partially or completely blocked under a condition where reverse transcription occurs and designed to have blocking of the primer function cleared under a condition where a polymerase chain reaction occurs.

As used herein, "kit" refers to a unit providing portions to be provided (e.g., reagent, agent, label, manual and the like) generally in two or more sections. This form of a kit is preferred when a composition that should not be provided in a mixed state and is preferably mixed immediately before use for safety or the like is intended to be provided. Such a kit advantageously comprises an instruction or manual describing how the provided portions (e.g., agents) are used or how a reagent should be handled. When the kit is used herein as a reagent kit, the kit generally comprises an instruction describing how an agent, antibody and the like is used.

As used herein, "instruction" is a document that explains to a user the method of using the present invention. The instruction has an instruction for the reverse transcription template switching PCR and the method of using a reagent of the present invention. The instruction may also have instructions for a method of use (screening method). The instruction is prepared in accordance with a format defined by a regulatory authority of the country in which the present invention is practiced, with an explicit description showing approval by the regulatory authority. The instruction is a so-called package insert, which can be provided in paper media or in a form such as electronic media (e.g., web sites provided on the Internet or emails).

A reagent required for a polymerase chain reaction comprised in the kit of the present invention does not need to comprise a primer. A primer may be included in the kit of the present invention or provided separately. Those skilled in the art can design and manufacture a suitable primer based on the target RNA or outsource the manufacture to a primer manufacturer. The modified oligonucleotide primer is used as a reverse primer used in the kit of the present invention. If the sequence of the 5' terminus side of the template RNA is unknown, a template switching oligonucleotide or a 5' anchor oligonucleotide primer comprising at least a part of an anchor sequence comprised in a template switching oligonucleotide is used as a forward primer that is used in the kit of the present invention. If the sequence of the 5' terminus side of the template RNA is known, a template switching oligonucleotide or a 5' anchor oligonucleotide primer, or a primer designed based on the known sequence of the 5' terminus side can be used as a forward primer that is used in the kit of the present invention.

In one embodiment, a reagent required for template switching in the kit of the present invention can comprise a template switching oligonucleotide. In another embodiment, a reagent required for a polymerase chain reaction in the kit of the present invention can, but does not need to comprise a 5' anchor oligonucleotide primer comprising at least a part of an anchor sequence comprised in a template switching oligonucleotide. As demonstrated in the Examples herein, a template switching oligonucleotide (TS-Oligo) can also unexpectedly function as a forward primer in PCR amplification. Therefore, a reagent required for a polymerase chain reaction can be free of the 5' anchor oligonucleotide primer or comprise a smaller amount than an amount that is commonly used.

Surprisingly, PCR amplification with high specificity was able to be achieved even by adding only the modified oligonucleotide primer without adding a reverse transcription primer to perform reverse transcription PCR. Although not wishing to be bound by any theory, a part of a modified oligonucleotide primer does not have the function blocked at the time of a reverse transcription reaction, so that a part of the modified oligonucleotide primer whose function is not blocked can function as a reserve transcription primer, or a function of a modified oligonucleotide primer is partially blocked at the time of a reverse transcription reaction, so that the modified oligonucleotide primer whose function is partially blocked can function as a reverse transcription primer in a limited capacity. Therefore in some embodiment, a reagent required for reverse transcription does not need to comprise an oligonucleotide primer that initiates reverse transcription. Even if it is comprised, the oligonucleotide primer that initiates reverse transcription used can be contained at a smaller amount than an amount that is commonly used. In some embodiments, the final concentration of the oligonucleotide primer that initiates reverse transcription to be used is, for example, about 40 nM or less, preferably about 20 nM or less, about 10 nM or less, about 2.5 nM or less, about 2.0 nM or less, about 0.63 nM or less, about 0.2 nM or less, about 0.16 nM or less, about 0.02 nM or less, about 2.0 pM or less, about 0.2 pM or less, or about 0.02 pM or less. In another embodiment, the oligonucleotide primer that initiates reverse transcription to be used is used at a mole ratio of about 1:10 or less relative to a modified oligonucleotide primer, preferably about 1:20 or less, about 1:40 or less, about 1:160 or less, about 1:200 or less, about 635:1 or less, about 2000:1 or less, about 2500:1 or less, about 20,000:1 or less, about 200,000:1 or less, about 2,000,000:1 or less, or about 20,000,000:1 or less.

The modified oligonucleotide primer used in the kit of the present invention has been described above in detail.

The present invention also provides a composition for amplifying at least a part of a region of a target RNA, comprising a modified oligonucleotide primer, wherein the modified oligonucleotide primer is designed to have a primer function that is partially blocked under a condition where reverse transcription occurs and designed to have the blocking of the primer function cleared under a condition where a polymerase chain reaction occurs, wherein a part of the modified oligonucleotide primer whose primer function has not been blocked functions as an oligonucleotide primer that starts reverse transcription by hybridizing to a template RNA. The composition of the present invention can be used in one-step reverse transcription PCR or one-step reverse transcription template switching PCR. As discussed above, a modified oligonucleotide primer used in the composition of the present invention can also function as an oligonucleotide primer that starts reverse transcription. Thus, an oligonucleotide primer that starts reverse transcription is not needed or used at a smaller amount than an amount that is commonly used in one-step reverse transcription PCR or one-step reverse transcription template switching PCR using the composition of the present invention. The modified oligonucleotide primer used in the composition of the present invention has been described above in detail.

Descriptions in all publications including reference literatures such as scientific literatures, patents, and patent applications cited herein are incorporated herein by reference to the same extent that the entirety of each document is specifically described.

The present invention has been described above with preferred embodiments to facilitate understanding. The present invention is described below based on Examples. The aforementioned description and the following Examples are not provided to limit the present invention, but for the sole purpose of exemplification. Thus, the scope of the present invention is not limited by the embodiments and Examples specifically described herein and is limited only by the scope of claims.

While the present invention is explained hereinafter in further detail with the following Examples, the present invention is not restricted in any way by the following Examples and the like.

### [Examples]

The following reagents were used.

**[Table 1]**

| **Reagent** | | **Manufacturer** |
|---|---|---|
| Kit | PrimeScriptIIHighFidelity Onestep RT-PCR kit | TaKaRa |
| Total RNA | **Total RNA purified from mouse T cells (β immobilized)** | |
| Inhibitor | RNasin Plus RNase Inhibitor {40U/µl) | Promega |
| | RNase Inhibitor (Cloned) (40 U/µL) | Ambion |
| | SUPERaseIn RNase Inhibitor (20 U/µL) | Ambion |
| SS 4 | SuperScriptIVReverse Transcriptase(200U/µl) (=SS4) | Invitrogen |
| Primer | CleanAmp™ Precision Primers (Block primer) RT primer | TriLink |
| TS-Oligo | Template switch oligo**(3 bases of 3' are RNA)** | |

The sequences of oligonucleotides used are the following:
Block primer: GAGGGTAGCCTTTTGTTTGTTTGCAATCTC (SEQ ID NO: 1)
RT primer: AAGCACACGAGGGTAGCCTTTTGTTTGTTTGCAA (SEQ ID NO: 2)
Template switch Oligo (3 bases of 3' are RNA): AAGCAGTGGTATACCCGCAGAGTACATrGrGrG (SEQ ID NO: 3).

The block primer and RT primer are reverse primers that are specific to the constant region of a mouse TCRβ chain. The full length is designed to hybridize to a TCRβ mRNA. The RT primer is designed to be nested on the 3' side by 4 bases. The RT primer has the 5' side extended by 8 bases to enhance the affinity. The constant region of an mRNA encoding a TCRβ chain to the 5' terminus can be amplified by using such primers and performing reverse transcription template switching PCR. The amplified region includes an untranslated region or reconstituted VDJ comprising an antigen recognition site and the like. Thus, a cDNA library of untranslated regions and antigen recognition sites of a TCRβ chain can be constructed by using the total RNA collected from a T cell population as a template and performing reverse transcription template switching PCR with such primers. If a single cell of T cell sorted by a cell sorter or the like is directly used as a template (RNA in the cells would be the template), the antigen recognition site of a TCRβ chain of an individual cell can be specifically amplified and sequenced.

In this test, reverse transcription template switching PCR was performed in one step. Typically, a reaction mixture with the composition in the following Table was used.

**[Table 2]**

| | | volume (µl) | final conc | |
|---|---|---|---|---|
| * | 2xone-step High Fidelity buffer | 5 | 1x | |
| * | PrimeScript II RT Enzyme mix (50x - x 1/1600) | 0.2 | 1/1600x | #1 |
| * | (12.5x)PrimeSTAR GXL for 1 step RT-PCR | 0.8 | 1x | #1 |
| | RT primer (0.5nM) | 0.4 | 0.02nM | |
| | CleanAmp™ Precision Primers (10µM) | 0.4 | 0.4 µM | |
| | Template switch oligo (10µM) | 0.4 | 0.4 µM | |
| | SS 4 (500/µl) | 0.5 | 2.5U/µl | |
| | Rnasein RNaseInhibitor | 0.1 | 0.4U/µl | |
| | Rnase inhibitor (cloned) | 0.1 | 0.4U/µl | |
| | SUPERase inhibitor | 0.1 | 0.2U/µl | |
| | Total RNA | 0.5 | 33.75pg/µl | |
| | H₂O | 1.5 | | |
| | Total | 10 | | |

| | | | | |
|---|---|---|---|---|
| * is Included in the PrimerScript II HighFidelity Pnesep RT PCT Kit #1: final amount in the manual is ×1. | | | | |

Typically, the following thermal cycling conditions were used.

**[Table 3]**

| | | | | | |
|---|---|---|---|---|---|
| | 95°C | 98°C | | | |
| | 0:05:00 | 0:00:10 | 60°C | | |
| 45°C | | | 0:00:06 | 60°C | |
| 0:30:00 | | | | 0:05:00 | 4°C |
| | | | | | ∞ |
| 1cycle | | 44 cycle | | 1cycle | |

| | | | | | |
|---|---|---|---|---|---|
| **Ramp Rate 6°C/s | | | | | |

The composition of a reaction solution and thermal cycling conditions were partially changed when appropriate depending on the test.

### [Test Example 1]

One-step reverse transcription template switching PCR was performed under the following total RNA concentration, block primer concentration, and RT primer concentration conditions.

**[Table 4]**

| | **Total RNA conc. (pg/µl)** | **Block primer (µM)** | **RT primer (µM)** |
|---|---|---|---|
| 1 | 34 | 0 | 0.4 |
| 2 | 34 | 0.4 | 0.04 |
| 3 | 34 | 0.4 | 0.01 |
| 4 | 34 | 0.4 | 0.0025 |
| 5 | 34 | 0.4 | 0,00063 |
| 6 | 34 | 0.4 | 0.00016 |
| 7 | 3.4 | 0.4 | 0.04 |
| 8 | 3.4 | 0.4 | 0.01 |
| 9 | 3.4 | 0.4 | 0.0025 |
| 10 | 3.4 | 0.4 | 0.00063 |
| 11 | 3.4 | 0.4 | 0.00016 |

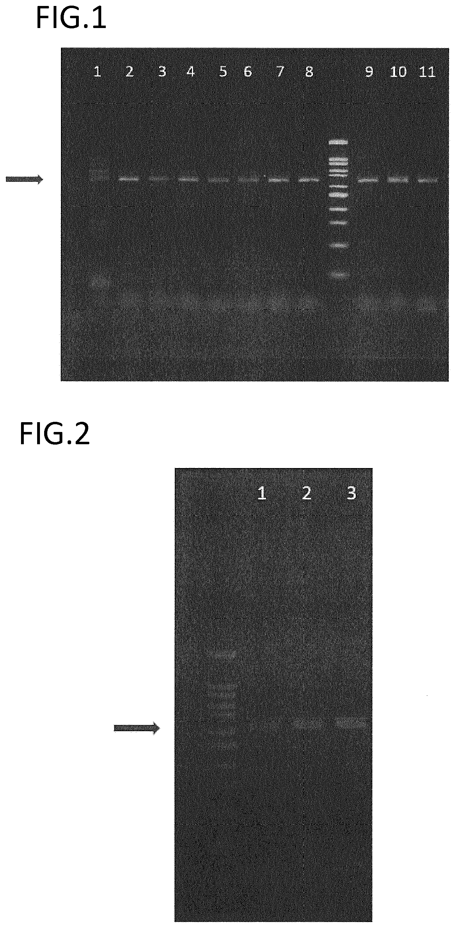

The results are shown in Figure 1. When a block primer was not used, a large number of non-specific bands were detected (lane 1), but the non-specific bands disappeared by adding a block primer (lanes 2 to 11). Specific amplification of a TCRβ chain was observed at RT primer concentrations of 0.04 µM to 0.00016 µM. While minor non-specific bands were observed at relatively high RT primer concentrations (0.4 µM and the like), this was able to be suppressed by reducing the RT primer concentration.

### [Test Example 2]

One-step reverse transcription template switching PCR was performed under the following cell count, block primer concentration, and RT primer concentration conditions. The number of PCR cycles was 48.

**[Table 5]**

| | **Number of cells** | **Block primer (µM)** | **RT primer (µM)** |
|---|---|---|---|
| 1 | 30 | 0.4 | 0.02 |
| 2 | 30 | 0.4 | 0.002 |
| 3 | 30 | 0.4 | 0 |

The results are shown in Figure 2. Specific amplification of a TCRβ chain was observed under any of the conditions. Specific amplification of a TCRβ chain was observed without adding an RT primer (lane 3). As a result of one-step reverse transcription template switching PCR under the same conditions as above by changing the cell count to 10 and the block primer to CleanAmp™ Turbo Primers (TriLink), specific amplification of a TCRβ chain was similarly observed.

### [Test Example 3]

One-step reverse transcription template switching PCR was performed after directly adding a reaction solution to a single cell of mouse T cell in one step. The number of PCR cycles was 56.

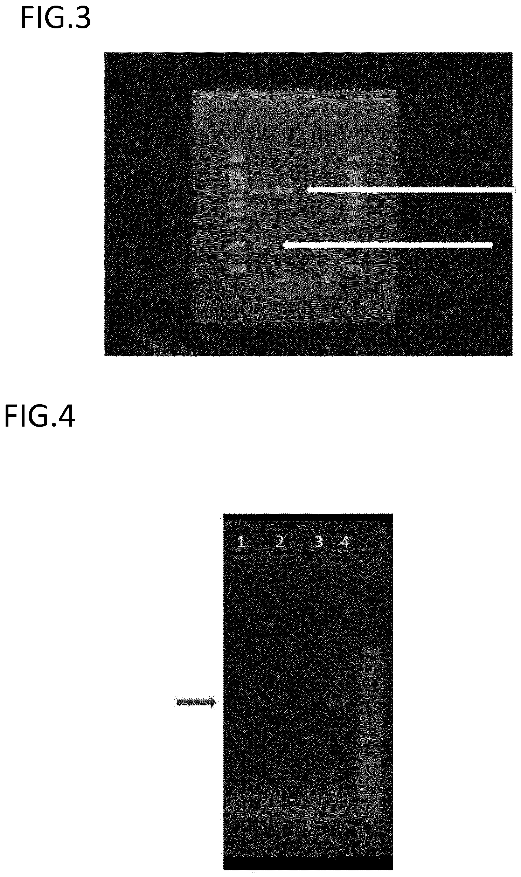

The results are shown in Figure 3. Cells with only the full length of a TCRβ chain amplified, and cells with the full length and fragment of a TCRβ chain amplified were observed. Despite the high number of cycles at 56, non-specific amplification was not observed.

### [Test Example 4]

The number of PCR amplification cycles was changed to various numbers (number of cycles: 38, 40, 42, and 44). The block primer concentration was 0.4 µm, and the RT primer concentration was 0.002 nM.

The results are shown in Figure 4 (lane 1: 38, lane 2: 40, lane 3: 42, and lane 4: 44). A specific band of a TCRβ chain was observed at 44 cycles.

### [Test Example 5]

One-step reverse transcription template switching PCR was performed under the following total RNA concentration, block primer concentration, and RT primer concentration conditions. The number of PCR cycles was 42.

**[Table 6]**

| | **Total RNA conc. (pg/µl)** | **Block primer (µM)** | **RT primer** |
|---|---|---|---|
| 1 | 3.4 | 0 | 0.4 µM |
| 2 | 3.4 | 0.2 | 0.2 µM |
| 3 | 3.4 | 0.4 | 0.02 µM |
| 4 | 3.4 | 0.4 | 2 nM |
| 5 | 3.4 | 0.4 | 0.2 nM |
| 6 | 3.4 | 0.4 | 0.02 nM |
| 7 | 3.4 | 0.4 | 2 pM |
| 8 | 3.4 | 0.4 | 0.2 pM |
| 9 | 3.4 | 0.4 | 0.02 pM |
| 10 | 3.4 | 0.4 | 0 |

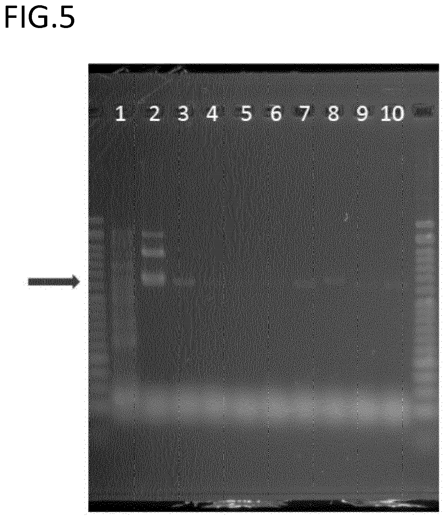

The results are shown in Figure 5. When a block primer was not used, a large number of smeared non-specific bands were detected (lane 1), but the non-specific bands disappeared by adding a block primer (lanes 2 to 10). While minor non-specific bands remained at a relatively high RT primer concentration (0.2 µM), this was able to be suppressed by reducing the RT primer concentration. Specific amplification of a TCRβ chain was observed without adding an RT primer (lane 10).

### [Test Example 6]

In this Test Example, an antigen recognition site of a TCRα chain was specifically amplified from a single regulatory T cell.

The following reagents were used.

**[Table 7]**

| Reagent | | Manufacturer |
|---|---|---|
| Kit | PrimeScript II HighFidelity Onestep RT-PCR kit | TaKaRa |
| Total RNA | Total RNA purified from mouse T cells (βimmobilized) | |
| Inhibitor | RNasin Plus RNase Inhibitor (40U/ul) | Promega |
| | RNase Inhibitor (Cloned) (40 U/uL) | Ambion |
| | SUPERaseIn RNase Inhibitor (20 U/uL) | Ambion |
| SS 4 | superscriptIVReverse Transcriptase(200U/ul) (=SS4) | Invitrogen |
| Primer | CleanAmp™ Precision Primers (Block primer) | TriLink |
| | RT primer | |
| TS-Oligo | Template switch oligo(first from the 3' terminus is LNA, second and third are RNAs) | |

The sequences of oligonucleotides used are the following:
Block primer: GAGGATCTTTTAACTGGTACACAGCAGGTTCTG (SEQ ID NO: 4)
RT primer: CGG TGA ACA GGC AGA GGG TG (SEQ ID NO: 5)
Template switch Oligo (first from the 3' terminus is LNA, second and third are RNAs) : AAGCAGTGGTATACCCGCAGAGTACATrGrG(L)G (SEQ ID NO: 3).

The block primer and RT primer are reverse primers that are specific to the constant region of a mouse TCRα chain. The constant region of an mRNA encoding a TCRα chain to the 5' terminus can be amplified by using such primers and performing reverse transcription template switching PCR. The amplified region includes an untranslated region or reconstituted VDJ comprising an antigen recognition site and the like. Thus, a cDNA library of untranslated regions and antigen recognition sites of a TCRβ chain can be constructed by using the total RNA collected from a T cell population as a template and performing reverse transcription template switching PCR with such primers. If a single cell of T cell sorted by a cell sorter or the like is directly used as a template (RNA in cells would be the template), the antigen recognition site of a TCRα chain of an individual cell can be specifically amplified and sequenced.

In this test, template switching RT-PCR was performed in one step. Typically, a reaction mixture with the composition in the following Table was used.

**[Table 8]**

| | | volume (µl) | final conc | |
|---|---|---|---|---|
| * | 2xone-step High Fidelity buffer | 5 | 1x | |
| * | (12.5x)PrimeSTAR GXL for 1 step RT-PCR | 0.8 | 1x | #1 |
| | RT primer (5nM) | 0.4 | 0.2nM | |
| | CleanAmp™ Precision Primers (10uM) | 0.4 | 0.4 µM | |
| | Template switch oligo (10uM) | 0.4 | 0.4 µM | |
| | SS 4 (50U/ul) | 0.5 | 2.5U/µl | |
| | Rnasein RNaseInhibitor | 0.1 | 0.4U/µl | |
| | Rnase inhibitor(cloned) | 0.1 | 0.4U/µl | |
| | SUPERase inhibitor | 0.1 | 0.2U/µl | |
| | Single cell (Treg) | | | |
| | H₂O | 2.2 | | |
| | Total | 10 | | |

Typically, the following thermal cycling conditions were used.

**[Table 9]**

| | | | | | | |
|---|---|---|---|---|---|---|
| | 95°C | 98°C | | | | |
| | 0:05:00 | 0:00:10 | | 68°C | | |
| 45°C | | | 60°C | 0:00:04 | 60°C | |
| 0:30:00 | | | 0:00:02 | | 0:05:00 | 4°C |
| | | | | | | ∞ |
| 1 cycle | | 56 cycle | | | 1 cycle | |

| | | | | | | |
|---|---|---|---|---|---|---|
| **Ramp Rate 6°C/s | | | | | | |

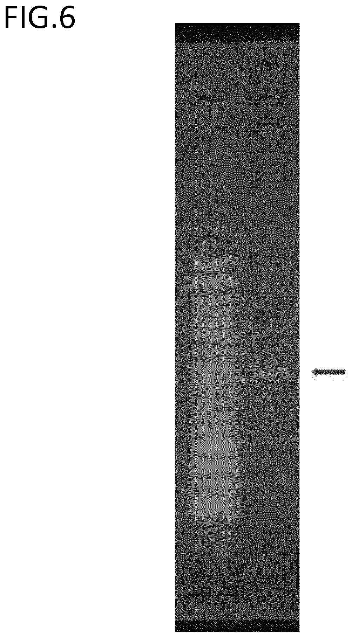

As a result of confirmation with electrophoresis after purification of the amplicon with AMpure beads, specific amplification of a TCRα chain was observed as a single band (Figure 6). Since a TCRα chain has a different sequence for each cell, observation of a single band shows that the method of the present invention is a method that is capable of amplification from a single cell (single regulatory T cell in this Text Example).

While the present invention has been explained while emphasizing the preferred embodiments, it is evident to those skilled in the art that the preferred embodiment can be modified. The present invention is intended to be practicable by a method other than those described in detail herein. Therefore, the present invention includes all modifications that are encompassed within the spirit and scope of the appended "Claims".

The content described in all of the publications including the patents and patent applications discussed herein are incorporated herein by reference to the same extent that the entirety thereof is explicitly described herein. The present invention claims priority to Japanese Patent Application No. 2016-125007 filed on June 23, 2016, which is incorporated herein to the same extent that the entirety thereof is explicitly described herein.

### [Industrial Applicability]

The present invention is expected to perform reverse transcription template switching PCR with high specificity in one step. In particular, even if the number of copies of a template RNA is low and the number of PCR cycles is high, a specific PCR product can be expected to be amplified while suppressing side reactions.

## Claims

1. A method of amplifying at least a part of a region of a target RNA, the method comprising the steps of:
a) mixing the target RNA, a reagent required for reverse transcription, a reagent required for template switching, and a reagent required for a polymerase chain reaction and subjecting the mixture to a condition under which reverse transcription occurs to provide a cDNA comprising a nucleic acid sequence corresponding to the target RNA and a template switching oligonucleotide; and
b) subjecting the cDNA obtained in step a) to a condition under which a polymerase chain reaction occurs to amplify at least a part of a region of the cDNA;
wherein the reagent required for a polymerase chain reaction comprises a modified oligonucleotide primer designed to have a primer function that is partially or completely blocked in step a) and designed to have blocking of the primer function cleared in step b).

2. A method of producing a nucleic acid sample that is amplified based on at least a part of a region of a target RNA, the method comprising the steps of:
a) mixing the target RNA, a reagent required for reverse transcription, a reagent required for template switching, and a reagent required for a polymerase chain reaction and subjecting the mixture to a condition under which reverse transcription occurs to provide a cDNA comprising a nucleic acid sequence corresponding to the target RNA and a template switching oligonucleotide; and
b) subjecting the cDNA obtained in step a) to a condition under which a polymerase chain reaction occurs;
wherein the reagent required for a polymerase chain reaction comprises a modified oligonucleotide primer designed to have a primer function that is partially or completely blocked in step a) and designed to have blocking of the primer function cleared in step b).

3. The method of claim 1 or 2, wherein the reagent required for a polymerase chain reaction optionally comprises a 5' anchor oligonucleotide primer comprising at least a part of an anchor sequence comprised in the template switching oligonucleotide.

4. The method of claim 3, wherein the reagent required for a polymerase chain reaction does not comprise the 5' anchor oligonucleotide primer.

5. The method of any one of claims 1 to 4, wherein the reagent required for reverse transcription comprises an oligonucleotide primer that initiates reverse transcription, and the oligonucleotide primer that initiates reverse transcription is comprised in the mixture at a final concentration of about 40 nM or less, or at a mole ratio of about 1:10 or less relative to the modified oligonucleotide primer.

6. The method of any one of claims 1 to 5, wherein the modified oligonucleotide primer has one or more complementary regions on a sequence of the same modified oligonucleotide primer, and has a turn structure by the complementary regions or comprises a thermolabile modifying group before initial thermal denaturation of PCR.

7. The method of any one of claims 1 to 6, wherein the modified oligonucleotide primer comprises a nucleotide sequence that is complementary to a partial sequence of a template RNA.

8. The method of claim 7, wherein a part of the modified oligonucleotide primer whose primer function has not been blocked functions as an oligonucleotide primer that initiates reverse transcription by hybridizing to the template RNA.

9. A kit for amplifying at least a part of a region of a target RNA, the kit comprising:
i) a reagent required for reverse transcription;
ii) a reagent required for template switching;
iii) a reagent required for a polymerase chain reaction using a modified oligonucleotide primer; and
iv) optionally a user manual;
**characterized in that** the reagents of i) to iii) and the modified oligonucleotide primer are all mixed in a reaction system as of the initiation of a reaction, wherein the modified oligonucleotide primer is designed to have a primer function that is partially or completely blocked under a condition where reverse transcription occurs and designed to have blocking of the primer function cleared under a condition where a polymerase chain reaction occurs.

10. The kit of claim 9, wherein the reagent required for template switching comprises a template switching oligonucleotide, and the reagent required for a polymerase chain reaction optionally comprises a 5' anchor oligonucleotide primer comprising at least a part of an anchor sequence comprised in the template switching oligonucleotide.

11. The kit of claim 10, wherein the reagent required for a polymerase chain reaction does not comprise the 5' anchor oligonucleotide primer.

12. The kit of any one of claims 9 to 11, **characterized in that** the reagent required for reverse transcription comprises an oligonucleotide primer that initiates reverse transcription, and the oligonucleotide primer that initiates reverse transcription is used at a final concentration of about 40 nM or less, or at a mole ratio of about 1:10 or less relative to the modified oligonucleotide primer.

13. The kit of any one of claims 9 to 12, wherein the modified oligonucleotide primer has one or more complementary regions on a sequence of the same modified oligonucleotide primer, and has a turn structure by the complementary regions or comprises a thermolabile modifying group before initial thermal denaturation of PCR.

14. The kit of any one of claims 9 to 13, wherein the modified oligonucleotide primer comprises a nucleotide sequence that is complementary to a partial sequence of a template RNA.

15. The kit of claim 14, wherein a part of the modified oligonucleotide whose primer function has not been blocked functions as an oligonucleotide primer that initiates reverse transcription by hybridizing to the template RNA.

16. A composition for amplifying at least a part of a region of a target RNA, comprising a modified oligonucleotide primer, wherein the modified oligonucleotide primer is designed to have a primer function that is partially blocked under a condition where reverse transcription occurs and designed to have the blocking of the primer function cleared under a condition where a polymerase chain reaction occurs, wherein a part of the modified oligonucleotide primer whose primer function has not been blocked functions as an oligonucleotide primer that initiates reverse transcription by hybridizing to a template RNA.

17. The composition of claim 16, wherein the modified oligonucleotide primer has one or more complementary regions on a sequence of the same modified oligonucleotide primer, and has a turn structure by the complementary regions or comprises a thermolabile modifying group before initial thermal denaturation of PCR.

18. The composition of claim 16 or 17, wherein the composition is used in one-step reverse transcription template switching PCR.
